# EUROPEAN PATENT APPLICATION

(11) **EP 3 533 859 A1**
(43) Date of publication of application: **04.09.2019**
(21) Application number: 19156263.6
(22) Date of filing: 08.02.2019
(51) Int. Cl.: C11D 3/386

(54) **CLEANING COMPOSITIONS**

(30) Priority: 28.02.2018 EP 18159331
(71) Applicant: The Procter & Gamble Company, Cincinnati, OH 45202 (US)
(72) Inventor: LANT, Neil Joseph, Newcastle upon Tyne, NE12 9TS (GB); SOUTER, Philip Frank, Newcastle upon Tyne, NE12 9TS (GB); VASQUEZ VALDIVIESO, Montserrat Guadalupe, Newcastle upon Tyne, NE12 9TS (GB)
(74) Representative: P&G Patent Belgium UK

(57) **Abstract**

Cleaning compositions that include particular glycogen- debranching enzymes, second amylase and cleaning adjunct and methods of making such cleaning compositions and methods of using such cleaning compositions to provide starchy soil removal even from complex stains.

## Description

### REFERENCE TO A SEQUENCE LISTING

This application contains a Sequence Listing in computer readable form, which is incorporated herein by reference.

### FIELD OF THE INVENTION

The present disclosure relates to cleaning compositions comprising certain isoamylase enzymes capable of breaking alpha-1,6-glycoside linkages (glycogen debranching enzymes), and mixtures thereof. The present invention also relates to methods of making and using such cleaning compositions.

### BACKGROUND OF THE INVENTION

The laundry detergent formulator is constantly aiming to improve the performance of detergent compositions. Starchy soils comprise polymeric carbohydrate consisting of glucose units joined by glycosidic bonds. A big proportion of many starch soils is amylopectin and this contains alpha-1,6 glycosidic bonds. One common ingredient used by detergent formulators to help remove starchy stains are amylase enzymes, typically having primary activity in attacking alpha-1,4 glycosidic bonds in the starch. However, starchy soils are often part of a more chemically complex soil, combined with other soil materials such as oils and proteins, making complete soil removal extremely challenging. Incomplete soil removal still results in spite of attempts to deliver improved starch breakdown, including by bringing together enzymes that attack alpha-1,4 and alpha-1,6 glycoside bonds, for example using amylases in combination with pullulanases. Although some specific pullulanases have glycogen-debranching activity, that activity is typically for the specific pullulan substrate for which isoamylases have no activity. Without being bound to theory, we believe that proteins, fats and other component in the complex soils stains are locking down starch soils. There is therefore still a need for improved cleaning compositions that provide stain removal benefits for cleaning starchy soils. There is also a need for improved cleaning of starchy soils, when the stains are part of complex mixtures with different soils, for example protein and/or fat in intimate mixture with the starch.

The present inventors have found that specific types of alpha-1,6 degrading enzymes, including those not used in nature for starch breakdown, are very effective.

Furthermore, the present inventors have surprisingly found that a combination of an amylase having primary activity for alpha-1,4 glycosidic bonds and a specific glycogen debranching enzyme can lead to superior stain removal in complex soils.

### SUMMARY OF THE INVENTION

The present invention relates to a cleaning composition comprising a glycogen-debranching enzyme having at least 60%, preferably at least 70% identity to SEQ ID NO:1; b) a second amylase, said second amylase having activity in breaking alpha-1,4- glycosidic bonds; and c) a cleaning adjunct.

The present invention also relates to a method of making a cleaning composition comprising mixing a glycogen-debranching enzyme with 60% identity to SEQ ID NO:1; b) an alpha amylase; and a cleaning adjunct.

The present invention also relates to a method of cleaning a surface, preferably a textile, where the method includes the step of mixing a cleaning composition as described herein with water to form an aqueous liquor and contacting a surface, preferably a textile, with the aqueous liquor in a washing step.

### DETAILED DESCRIPTION OF THE INVENTION

The components of the compositions and processes of the present disclosure are described in more detail below.

As used herein, the articles "a" and "an" when used in a claim, are understood to mean one or more of what is claimed or described. As used herein, the terms "include," "includes," and "including" are meant to be non-limiting. The compositions of the present invention can comprise, consist essentially of, or consist of, the components of the present invention.

The terms "substantially free of' or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1%, or less than 0.01%, or even 0%, by weight of the composition.

As used herein, the term "etheramine" includes the term "polyetheramine" and includes amines that have one or more ether groups.

Unless otherwise noted, all component or composition levels are in reference to the active portion of that component or composition, and are exclusive of impurities, for example, residual solvents or by-products, which may be present in commercially available sources of such components or compositions.

All temperatures herein are in degrees Celsius (°C) unless otherwise indicated. Unless otherwise specified, all measurements herein are conducted at 20°C and under the atmospheric pressure.

In all embodiments of the present disclosure, all percentages are by weight of the total composition, unless specifically stated otherwise. All ratios are weight ratios, unless specifically stated otherwise.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "alkoxy" is intended to include C1-C8 alkoxy and C1-C8 alkoxy derivatives of polyols having repeating units such as butylene oxide, glycidol oxide, ethylene oxide or propylene oxide.

As used herein, unless otherwise specified, the terms "alkyl" and "alkyl capped" are intended to include C1-C18 alkyl groups, or even C1-C6 alkyl groups.

As used herein, unless otherwise specified, the term "aryl" is intended to include C3-12 aryl groups.

As used herein, unless otherwise specified, the term "arylalkyl" and "alkaryl" are equivalent and are each intended to include groups comprising an alkyl moiety bound to an aromatic moiety, typically having C1-C18 alkyl groups and, in one aspect, C1-C6 alkyl groups.

The terms "ethylene oxide," "propylene oxide" and "butylene oxide" may be shown herein by their typical designation of "EO," "PO" and "BO," respectively.

As used herein, the term "cleaning and/or treatment composition" includes, unless otherwise indicated, granular, powder, liquid, gel, paste, unit dose, bar form and/or flake type washing agents and/or fabric treatment compositions, including but not limited to products for laundering fabrics, fabric softening compositions, fabric enhancing compositions, fabric freshening compositions, and other products for the care and maintenance of fabrics, and combinations thereof. Such compositions may be pre-treatment compositions for use prior to a washing step or may be rinse added compositions, as well as cleaning auxiliaries, such as bleach additives and/or "stain-stick" or pre-treat compositions or substrate-laden products such as dryer added sheets.

As used herein, "cellulosic substrates" are intended to include any substrate which comprises cellulose, either 100% by weight cellulose or at least 20% by weight, or at least 30 % by weight or at least 40 or at least 50 % by weight or even at least 60 % by weight cellulose. Cellulose may be found in wood, cotton, linen, jute, and hemp. Cellulosic substrates may be in the form of powders, fibers, pulp and articles formed from powders, fibers and pulp. Cellulosic fibers, include, without limitation, cotton, rayon (regenerated cellulose), acetate (cellulose acetate), triacetate (cellulose triacetate), and mixtures thereof. Typically, cellulosic substrates comprise cotton. Articles formed from cellulosic fibers include textile articles such as fabrics. Articles formed from pulp include paper.

As used herein, the term "maximum extinction coefficient" is intended to describe the molar extinction coefficient at the wavelength of maximum absorption (also referred to herein as the maximum wavelength), in the range of 400 nanometers to 750 nanometers.

As used herein "average molecular weight" is reported as a weight average molecular weight, as determined by its molecular weight distribution; as a consequence of their manufacturing process, polymers disclosed herein may contain a distribution of repeating units in their polymeric moiety.

As used herein the term "variant" refers to a polypeptide that contains an amino acid sequence that differs from a wild type or reference sequence. A variant polypeptide can differ from the wild type or reference sequence due to a deletion, insertion, or substitution of a nucleotide(s) relative to said reference or wild type nucleotide sequence. The reference or wild type sequence can be a full-length native polypeptide sequence or any other fragment of a full- length polypeptide sequence. A polypeptide variant generally has at least about 70% amino acid sequence identity with the reference sequence, but may include 75% amino acid sequence identity within the reference sequence, 80% amino acid sequence identity within the reference sequence, 85% amino acid sequence identity with the reference sequence, 86% amino acid sequence identity with the reference sequence, 87% amino acid sequence identity with the reference sequence, 88% amino acid sequence identity with the reference sequence, 89% amino acid sequence identity with the reference sequence, 90% amino acid sequence identity with the reference sequence, 91% amino acid sequence identity with the reference sequence, 92% amino acid sequence identity with the reference sequence, 93% amino acid sequence identity with the reference sequence, 94% amino acid sequence identity with the reference sequence, 95% amino acid sequence identity with the reference sequence, 96% amino acid sequence identity with the reference sequence, 97% amino acid sequence identity with the reference sequence, 98% amino acid sequence identity with the reference sequence, 98.5% amino acid sequence identity with the reference sequence or 99% amino acid sequence identity with the reference sequence.

As used herein, the term "solid" includes granular, powder, bar and tablet product forms.

As used herein, the term "fluid" includes liquid, gel, paste, and gas product forms.

### Cleaning Composition

The present disclosure relates to cleaning and/or treatment compositions. The cleaning composition may be a laundry or hard surface cleaning compositions or additives. Examples of hard surface cleaning compositions include for example dishwashing detergents or additives for dish-washing which may be for automatic dishwashing or handwashing. The cleaning composition is preferably a dishwashing composition, preferably an automatic dishwashing detergent or a laundry composition (such as a heavy duty liquid or solid detergent composition).

The cleaning compositions may be in any suitable form. The composition can be selected from a liquid, solid, or combination thereof. As used herein, "liquid" includes free-flowing liquids, as well as pastes, gels, foams and mousses. Non-limiting examples of liquids include light duty and heavy duty liquid detergent compositions, fabric enhancers, detergent gels commonly used for laundry, bleach and laundry additives. Gases, e.g., suspended bubbles, or solids, e.g. particles, may be included within the liquids. A "solid" as used herein includes, but is not limited to, powders, agglomerates, and mixtures thereof. Non-limiting examples of solids include: granules, micro-capsules, beads, noodles, and pearlised balls. Beads, noodles and pearlized balls in particular may be additives for fabric treatment. Solid compositions may provide a technical benefit including, but not limited to, through-the-wash benefits, pre-treatment benefits, and/or aesthetic effects.

The cleaning composition may be in the form of a unitized dose article, such as a tablet or in pouch form. Such pouches typically include a water-soluble film, such as a polyvinyl alcohol water-soluble film, that at least partially encapsulates a composition. Suitable films are available from MonoSol, LLC (Indiana, USA). The composition can be encapsulated in a single or multi-compartment pouch. A multi-compartment pouch may have at least two, at least three, or at least four compartments. A multi-compartmented pouch may include compartments that are side-by-side and/or superposed. The composition contained in the pouch may be liquid, solid (such as powders), or combinations thereof.

### Glycogen-Debranching Enzyme

The glycogen debranching enzyme is a glycoside hydrolase. Preferably the glycogen-debranching enzyme belongs to EC 3.2.1.68. Preferably the glycogen-debranching enzyme degrades 1,6-glucosidic linkages, and most preferably shows distinct substrate specificity toward limit dextrins and phosphorylase limit dextrin. Preferably the glycogen-debranching enzyme is from family 13. The glycogen-debranching enzyme has at least 60% sequence identity to SEQ ID NO:1, preferably at least 70%, more preferably at least 75%, or at least 80% or at least 85% or at least 90% or at least 91, or at least 92, or at least 93, or at least 94 or or at least 95% identity to SEQ ID NO: 1.

Preferably the glycogen-debranching enzyme is a variant of SEQ ID NO: 1. The glycogen-debranching enzyme may be of any suitable origin such as yeasts, fungi and bacteria. Preferably however, they are bacterial. Suitable microbial sources are for example *Pseudomonas, Corynebacterium glutamicum* or *E. coli,* E. coli is preferred.

The glycogen-debranching enzyme may be used singly or in combination. They may be used in non-purified or purified form, irrespective of the methods of purification. They may be incorporated into the cleaning composition in liquid form or in the form of a particle (solid form), often known as an enzyme prill. They may be added into a cleaning composition via a premix with other enzymes such as other amylase, lipase, protease, cellulase, mannanase, pectate lyase, nuclease, cutinase, or mixtures thereof. The premix may be in liquid or solid form. Preferably the isoamylase is present in the cleaning composition of the invention in an amount at least 0.01 mg, preferably from about 0.05 to about 10, more preferably from about 0.1 to about 6, especially from about 0.2 to about 5 mg of active isoamylase/g of composition.

### Sequence ID NO: 1

MTQLAIGKPAPLGAHYDGQGVNFTLFSAHAERVELCVFDANGQEHRYDLPGHS
GDIWHGYLPDARPGLRYGYRVHGPWQPAEGHRFNPAKLLIDPCARQIDGEFKDNPLLH
AGHNEPDYRDNAAIAPKCVVVVDHYDWEDDAPPRTPWGSTIIYEAHVKGLTYLHPEIP
VEIRGTYKALGHPVMINYLKQLGITALELLPVAQFASEPRLQRMGLSNYWGYNPVAMF
ALHPAYACSPETALDEFRDAIKALHKAGIEVILDIVLNHSAELDLDGPLFSLRGIDNRSYY
WIREDGDYHNWTGCGNTLNLSHPAVVDYASACLRYWVETCHVDGFRFDLAAVMGRT
PEFRQDAPLFTAIQNCPVLSQVKLIAEPWDIAPGGYQVGNFPPLFAEWNDHFRDAARRF
WLHYDLPLGAFAGRFAASSDVFKRNGRLPSAAINLVTAHDGFTLRDCVCFNHKHNEAN

GEENRDGTNNNYSNNHGKEGLGGSLDLVERRRDSIHALLTTLLLSQGTPMLLAGDEHG
HSQHGNNNAYCQDNQLTWLDWSQASSGLTAFTAALIHLRKRIPALVENRWWEEGDGN
VRWLNRYAQPLSTDEWQNGPKQLQ
ILLSDRFLIAINATLEVTEIVLPAGEWHAIPPFAGEDNPVITAVWQGPAHGLCVFQ
R

### Second Amylase:

Suitable alpha-amylases include those of bacterial or fungal origin. Chemically or genetically modified mutants (variants) are included. A preferred alkaline alpha-amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) variants described in WO 94/02597, WO 94/18314, WO96/23874 and WO 97/43424, especially the variants with substitutions in one or more of the following positions versus the enzyme listed as SEQ ID NO: 2 in WO 96/23874: 15, 23, 105, 106, 124, 128, 133, 154, 156, 181, 188, 190, 197, 202, 208, 209, 243, 264, 304, 305, 391, 408, and 444.
(b) variants described in WO 96/23873, WO00/60060, WO06/002643 and WO2017/192657, especially the variants with one or more substitutions in the following positions versus the AA560 enzyme listed as SEQ ID NO: 12 in WO 06/002643:
   26, 30, 33, 82, 37, 106, 118, 128, 133, 149, 150, 160, 178, 182, 186, 193, 203, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(c) variants exhibiting at least 90% identity with SEQ ID No. 4 and described in WO06/002643, the wild-type enzyme from Bacillus SP722, especially variants with deletions in the 183 and 184 positions and variants described in WO 00/60060, or variants disclosed in WO2018/144399, both of which are incorporated herein by reference.
(d) variants exhibiting at least 95% identity with the wild-type enzyme from Bacillus sp.707 (SEQ ID NO:7 in US 6,093, 562), and described therein, especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations.
(e) variants described in WO 09/149130, preferably those exhibiting at least 90% identity with SEQ ID NO: 1 or SEQ ID NO:2 in WO 09/149130, the wild-type enzyme from Geobacillus Stearophermophilus or a truncated version thereof.
(f) variants exhibiting at least 89% identity with SEQ ID NO:1 in WO2016/091688, and as described in WO2016/091688 especially those comprising deletions at positions H183+G184 and additionally one or more mutations at positions 405, 421, 422 and/or 428.
(g) variants exhibiting at least 60% amino acid sequence identity with the "PcuAmyl α-amylase" from Paenibacillus curdlanolyticus YK9 (SEQ ID NO:3 in WO2014099523) or as described in WO2014099523.
(h) variants exhibiting at least 60% amino acid sequence identity with the "CspAmy2 amylase" from Cytophaga sp. (SEQ ID NO:6 in WO2014164777), for example a variant as described in WO2014164777.
(i) variants exhibiting at least 85% identity with AmyE from Bacillus subtilis (SEQ ID NO:1 in WO2009149271), for example a variant as described in WO2009149271.
(j) variants exhibiting at least 90% identity with the wild-type amylase from Bacillus sp. KSM-K38 with accession number AB051102.
(k) variants exhibiting at least 80% identity with the mature amino acid sequence of AAI10 from Bacillus sp (SEQ ID NO:7 in WO2016180748), for example a variant as described in WO2016/180748.
(l) variants exhibiting at least 80% identity with the mature amino acid sequence of Alicyclobacillus sp. amylase (SEQ ID NO:8 in WO2016180748), for example a variant as described in WO2016/180748.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL®, ATLANTIC®, INTENSA®, EVEREST® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS®, POWERASE®, PREFERENZ S® series (including PREFERENZ S1000® and PREFERENZ S2000® and PURASTAR OXAM® (DuPont., Palo Alto, California) and KAM® (Kao, 14-10 Nihonbashi Kayabacho, 1-chome, Chuo-ku Tokyo 103-8210, Japan). In one aspect, suitable amylases include ATLANTIC®, STAINZYME®, POWERASE®, INTENSA®, STAINZYME PLUS®, EXPEDASE® and mixtures thereof.

Particularly preferred second amylase is selected from amylases derived from AA560 alpha amylase endogenous to Bacillus sp. DSM 12649, preferably comprising one or more of the following mutations: R118K, D183*, G184*, N195F, R320K, and/or R458K.

### Cleaning Adjuncts

The cleaning compositions described herein comprises one or more cleaning adjuncts. Suitable adjuncts preferably comprise at least surfactant, preferably a surfactant system comprising a mixture of surfactants. Suitable adjuncts may include one or more of the following non-limiting list of ingredients: surfactant, surfactant system, fabric care benefit agent; detersive enzyme; deposition aid; rheology modifier; builder; chelant; bleach; bleaching agent; bleach precursor; bleach booster; bleach catalyst; bleach activator, encapsulated benefit agents, including where perfume is in the core; perfume; perfume loaded zeolite; starch encapsulated accord; polyglycerol esters; whitening agent; pearlescent agent; additional enzymes; enzyme stabilizing systems; scavenging agents including fixing agents for anionic dyes, chelant/complexing agents, and mixtures thereof; optical brighteners or fluorescers; polymer including but not limited to soil release polymer and/or soil suspension polymer and/or dye transfer inhibitor polymer; dispersants; antifoam agents; non-aqueous solvent; fatty acid; alkoxylated polyaryl/polyalkyl phenol, suds suppressors, e.g., silicone suds suppressors; cationic starches; scum dispersants; fabric shading dyes; colorants; opacifier; antioxidant; hydrotropes such as toluenesulfonates, cumenesulfonates and naphthalenesulfonates; color speckles; colored beads, spheres or extrudates; clay softening agents; anti-bacterial agents; quaternary ammonium compounds; and/or solvent or solvent systems comprising a mixture of solvents. Quaternary ammonium compounds may be particularly present in fabric enhancer compositions, such as fabric softeners, and comprise quaternary ammonium cations that are positively charged polyatomic ions of the structure NR₄⁺, where R is an alkyl group or an aryl group. Preferably the composition of the invention comprises a surfactant, or more preferably a surfactant system comprising a combination of surfactants. Preferably the composition of the invention comprises a cellulose polymer, in particular a modified cellulose polymer. Other preferred adjuncts comprise fabric shading agents as described below and/or an additional enzyme as described below, for example selected from lipases, nucleases, amylases, proteases, mannanases, pectate lyases, cellulases, cutinases, and mixtures thereof. The cleaning composition may comprise a cleaning cellulase.

### Surfactant system

The cleaning composition preferably comprises a surfactant system, preferably in an amount from about 1% to about 80%, or from 5% to about 60%, preferably from about 8 to about 50% more preferably from about 12% to about 40%, by weight of the cleaning composition, of a surfactant system. Suitable surfactants may be derived from natural and/or renewable sources.

The surfactant system preferably comprises a non-soap anionic surfactant, more preferably an anionic surfactant selected from the group consisting of linear alkyl benzene sulfonate, alkyl sulfate, alkyl alkoxy sulfate, especially alkyl ethoxy sulfate, paraffin sulfonate and mixtures thereof, preferably comprising linear alkyl benzene sulphonate. The surfactant system preferably further comprises a surfactant selected from the group consisting of nonionic surfactant, cationic surfactant, amphoteric surfactant, zwitterionic surfactant, and mixtures thereof. The surfactant system preferably comprises a nonionic surfactant; the nonionic surfactant may comprise an ethoxylated nonionic surfactant.

The most preferred surfactant system for the detergent composition of the present invention comprise from 1% to 40%, preferably 6% to 35%, more preferably 8% to 30% weight of the total composition of an anionic surfactant, preferably comprising linear alkyl benzene sulphonate optionally additionally comprising an alkyl alkoxy sulfate surfactant., and a nonionic surfactant, preferably an ethoxylated nonionic surfactant. Preferably the weight ratio of anionic surfactant to nonionic surfactant is from 200:1 to 1:2, more preferably from 100:1 to 1:1.

### Anionic surfactant

The non-soap anionic surfactant may comprise a sulphate or a sulphonate anionic surfactant or a mixture thereof, preferably linear alkylbenzene sulphonate, alkyl sulphate, alkoxylated alkyl sulphate or a mixture thereof, more preferably a mixture of linear alkylbenzene sulphonate and alkoxylated alkyl sulphate. Preferably, the ratio of linear alkylbenzene sulphonate to alkoxylated alkyl sulphate more preferably the ratio of linear alkylbenzene sulphonate to ethoxylated alkyl sulphate is from 1:2 to 20:1, preferably from 1.1:1 to 15:1, more preferably from 1.2:1 to 10:1, even more preferably from 1.3:1 to 5:1, most preferably from 1.4:1 to 3:1.

Preferably, the alkoxylated alkyl sulphate is an ethoxylated alkyl sulphate with an average degree of ethoxylation of between 0.5 and 7, preferably between 1 and 5, more preferably between 2 and 4, most preferably about 3. Alternatively, the non-soap surfactant comprises a mixture of one or more alkoxylated alkyl sulphates, preferably ethoxylated alkyl sulphates, and optionally an alkyl sulphate, the mixture having an average degree of ethoxylation of between 0.5 and 7, preferably between 1 and 5, more preferably between 2 and 4, most preferably about 3. The alkyl sulphate and/or alkoxylated alkyl sulphate preferably have an alkyl chain comprising on average between 8 and 18 carbon atoms, preferably between 10 and 16 carbons atoms, most preferably between 12 and 14 carbon atoms. Most preferably the alkoxylated alkyl sulphate is an ethoxylated alkyl chain comprising on average between 12 and 14 carbon atoms in its alkyl chain and has an average degree of ethoxylation of about 3. The alkyl chain of the alkoxylated alkyl sulphate surfactant may be linear or branched or a mixture thereof.

The linear alkylbenzene sulphonate may be a C₁₀-C₁₆ linear alkylbenzene sulphonate or a C₁₁-C₁₄ linear alkylbenzene sulphonate or a mixture thereof.

Exemplary linear alkylbenzene sulphonates are C₁₀-C₁₆ alkyl benzene sulfonic acids, or C₁₁-C₁₄ alkyl benzene sulfonic acids. By 'linear', we herein mean the alkyl group is linear. Alkyl benzene sulfonates are well known in the art.

Other suitable anionic detersive surfactants include alkyl ether carboxylates.

Suitable anionic detersive surfactants may be in salt form, suitable counter-ions include sodium, calcium, magnesium, amino alcohols, and any combination thereof. A preferred counter-ion is sodium.

### Nonionic surfactant

Preferably the nonionic surfactant is present in an amount from 0.1% to 12%, preferably 0.2% to 10%, most preferably 0.5% to 7%, most preferably from 1 to 3% by weight of the composition. Preferably the nonionic surfactant comprises a fatty alcohol ethoxylate.

Suitable alcohol ethoxylate nonionic surfactants include the condensation products of aliphatic alcohols with ethylene oxide. The alkyl chain of the aliphatic alcohol can either be straight or branched, substituted or unsubstituted. The starting alcohol can be naturally derived, e.g. starting from natural oils, or synthetically derived, e.g. alcohols obtained from for example oxo-, modified oxo- or Fischer-Tropsch processes. Examples of oxo-process derived alcohols include the Lial and Isalchem alcohols ex Sasol company and Lutensol alcohols ex BASF company. Examples of modified-oxo process derived alcohols include the Neodol alcohols ex Shell company. Fischer-Tropsch derived alcohols include Safol alcohols ex Sasol company. The alkoxylate chain of alcohol ethoxylates is made up solely of ethoxylate groups.

Preferably, the fatty alcohol ethoxylate has an average alkyl carbon chain length of between 5 and 30, preferably between 8 and 18, more preferably between 10 and 16, most preferably between 12 and 15.

Preferably, the fatty alcohol ethoxylate has an average degree of ethoxylation of between 0.5 and 20, preferably between 1 and 15, more preferably between 5 and 12, even more preferably between 6 and 10, most preferably between 7 and 8.

Suitable for use herein are the ethoxylated alcohol of the formula R(OC₂H₄)n OH, wherein R is selected from the group consisting of aliphatic hydrocarbon radicals containing from about 8 to about 22 carbon atoms and the average value of n is from about 5 to about 22. In one aspect, particularly useful materials are condensation products of C₉-C₁₆ alcohols with from about 5 to about 20 moles of ethylene oxide per mole of alcohol. In another aspect, particularly useful materials are condensation products of C₁₂-C₁₆ alcohols with from about 6 to about 9 moles of ethylene oxide per mole of alcohol.

Other non-limiting examples of nonionic surfactants may include: C12-C18 alkyl ethoxylates based on modified oxo alcohols, such as, NEODOL® nonionic surfactants from Shell; C12-C15 alkyl ethoxylates based on Fischer Tropsch Oxo alcohols, such as, SAFOL® nonionic surfactants from Sasol; C12-C18 alkyl ethoxylates based on natural or Ziegler alcohols, such as, Surfonic® nonionic surfactants from Huntsman; C14-C22 mid-chain branched alcohols ethoxylates, BAE*x*, wherein *x* is from 1 to 30.

Other suitable non-ionic surfactants for use herein include fatty alcohol polyglycol ethers, alkylpolyglucosides and fatty acid glucamides.

### Amphoteric surfactant

The surfactant system may include amphoteric surfactant, such as amine oxide. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the α carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that |n1 - n2| is less than or equal to 5, preferably 4, most preferably from 0 to 4 carbon atoms in at least 50 wt%, more preferably at least 75 wt% to 100 wt% of the mid-branched amine oxides for use herein. The amine oxide may further comprise two moieties, independently selected from a C1-3 alkyl, a C1-3 hydroxyalkyl group, or a polyethylene oxide group containing an average of from about 1 to about 3 ethylene oxide groups. Preferably the two moieties are selected from a C1-3 alkyl, more preferably both are selected as a C1 alkyl.

### Zwitterionic surfactant

Other suitable surfactants include betaines, such as alkyl betaines, alkylamidobetaine, amidazoliniumbetaine, sulfobetaine (INCI Sultaines) as well as the Phosphobetaine and preferably meets formula (I):

R¹-[CO-X(CH₂)ₙ]ₓ-N⁺(R²)(R₃)-(CH₂)ₘ-[CH(OH)-CH₂]_{y}-Y- (I)

wherein
R¹ is a saturated or unsaturated C6-22 alkyl residue, preferably C8-18 alkyl residue, in particular a saturated C10-16 alkyl residue, for example a saturated C12-14 alkyl residue;
X is NH, NR⁴ with C1-4 Alkyl residue R⁴, O or S,
n a number from 1 to 10, preferably 2 to 5, in particular 3,
x 0 or 1, preferably 1,
R², R³ are independently a C1-4 alkyl residue, potentially hydroxy substituted such as a hydroxyethyl, preferably a methyl.
m a number from 1 to 4, in particular 1, 2 or 3,
y 0 or 1 and
Y is COO, SO3, OPO(OR⁵)O or P(O)(OR⁵)O, whereby R⁵ is a hydrogen atom H or a C1-4 alkyl residue.

Preferred betaines are the alkyl betaines of the formula (Ia), the alkyl amido propyl betaine of the formula (Ib), the Sulfo betaines of the formula (Ic) and the Amido sulfobetaine of the formula (Id);

R¹-N⁺(CH₃)₂-CH₂COO⁻ (Ia)

R¹-CO-NH(CH₂)₃-N⁺(CH₃)₂-CH₂COO⁻ (Ib)

R¹-N⁺(CH₃)₂-CH₂CH(OH)CH₂SO₃- (Ic)

R¹-CO-NH-(CH₂)₃-N⁺(CH₃)₂-CH₂CH(OH)CH₂SO₃- (Id) in which R¹I as the same meaning as in formula I. Particularly preferred betaines are the Carbobetaine [wherein Y⁻=COO⁻ ], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines.

A preferred betaine is, for example, Cocoamidopropylbetaine.

### Fatty Acid

The surfactant may comprise a fatty acid, neutralised fatty acid soap or a mixture thereof. Preferably, the liquid laundry detergent composition may comprise less than 10%, preferably less than 8%, more preferably less than 5%, most preferably between 1% and 5% by weight of the liquid laundry detergent composition of fatty acid, neutralised fatty acid soap or a mixture thereof.

The neutralised fatty acid soap may be alkali metal neutralised, amine neutralised or a mixture thereof. The alkali metal may be selected from sodium, potassium, magnesium or a mixture thereof, preferably sodium. The amine is preferably an alkanolamine, preferably selected from monoethanolamine, diethanolamine, triethanolamine or a mixture thereof, more preferably monoethanolamine.

The fatty acid, neutralised fatty acid soap or mixture thereof may be selected from palm kernel fatty acid, coconut fatty acid, rapeseed fatty acid, neutralized palm kernel fatty acid, neutralized coconut fatty acid, neutralized rapeseed fatty acid, or mixture thereof, preferably neutralized palm kernel fatty acid.

### Fabric Shading Dye

The composition may comprise a fabric shading agent. Suitable fabric shading agents include dyes, dye-clay conjugates, and pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof. Preferred dyes are selected from azo, anthraquinone, triarylmethane and azine dyes and mixtures thereof, most preferably azo dyes. Preferered small molecule dyes comprise for example, Solvent Violet 13, Acid Violet 50, Acid Violet 51, Basic Violet 4, Direct Violet 9, Direct Violet 99, Direct Violet 66 and mixtures thereof. Most preferred are polymeric dyes wherein the polymer comprises a cellulose polymer, polyvinyl alcohol polymer, polyvinylpyrrolidone polymer or most preferably a polyalkoxylate polymer. Most preferred dyes comprise alkoxylated dyes, such as alkoxylated azo or anthraquinone or triarylmethane dyes. Alkoxylated azo dyes are preferred, particularly alkoxylated thiophene dyes, for example: 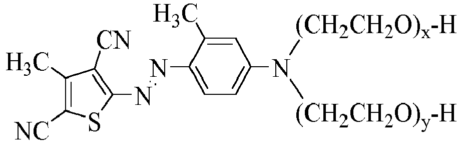 wherein the index values x and y are independently selected from 1 to 10.

### Chelant

The composition preferably comprises a complexing agent adjunct. A suitable chelant comprises a phosphonate chelant, for example selected from: 1-hydroxyethane-1,1-diphosphonic acid (HEDP); Diethylene triamine pentamethylene phosphonic acid (DTPMP, CW-Base); 2-phosphonobutane-1,2,4-tricarboxylic acid (PBTC); Amino trimethylene phosphonic acid (ATMP); Ethylenediamine tetramethylene phosphonic acid (EDTMP); Diethylenetriamine pentamethylene phosphonic acid (DTPMP); Aminotrimethylene phosphonic acid (ATMP); salts of the aforementioned materials; and any combination thereof.

Preferred complexing agents comprise an amino acid derivative complexing agent, preferably selected from one or more of the following, in any stereoisomer or mixture of stereoisomer form:
(i) methylglycinediacetic acid and salts thereof (MGDA)
(ii) L-glutamic acid, N,N-diacetic acid and salts thereof (GLDA) and
(iii) L-aspartic acid N,N-diacetic acid and salts thereof (ASDA)

Preferbly, the composition comprises from 0.1wt% to 10wt% methylglycinediacetic acid and salts thereof (MGDA)
It may be preferred to formulate the chelant/complexing agent in acid form. Alternatively, it may be preferred to formulate the amino acid derivative complexing agent in salt form, especially preferred is the sodium salt form.

Suitable MGDA salts are produced by BASF. Suitable GLDA salts are produced by Akzo Nobel and Showa Denko. Suitable ASDA salts are produced by Mitsubishi Rayon.

### Alkoxylated polyaryl/polyalkyl phenol

The compositions of the invention may comprise a polyaryl/polyalkyl phenol adjunt. A suitable alkoxylated polyaryl/polyalkyl phenol has the following structure: 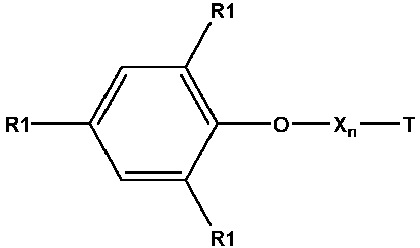 wherein R₁ is selected from linear of branched C₃-C₁₅ alkyl groups and aryl groups, X is selected from ethoxy or propoxy groups, n is from 2 to 70, T is selected from H, SO₃⁻, COO⁻ and PO₃²⁻

The alkoxylated polyaryl or alkoxylated polyalkyl phenol is preferably selected from groups (i) to (iv):
(i) Uncharged alkoxylated tristyrylphenols of the following structure: 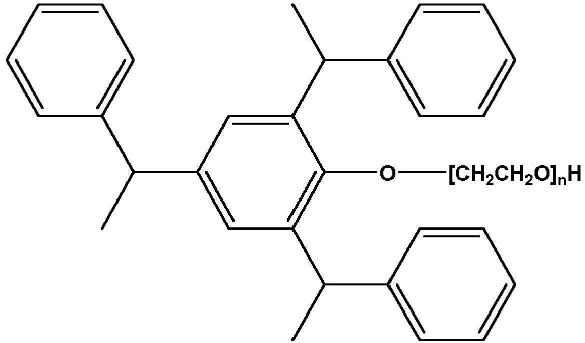 wherein n is selected from 2 to 70, more preferably n is selected from 10 to 54, most preferably n = 16 or 20.
(ii) Anionic alkoxylated tristyrylphenols of the following structure 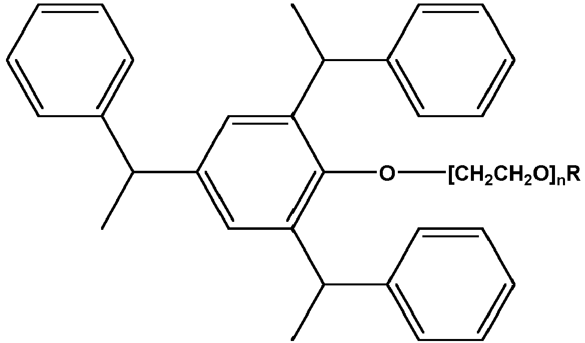 wherein R is selected from SO₃⁻, COO⁻ and PO₃²⁻, preferably selected from SO₃⁻ and COO⁻, wherein n is selected from 2 to 54.
(iii) Uncharged alkoxylated tri(n-butyl)phenols of the following structure: 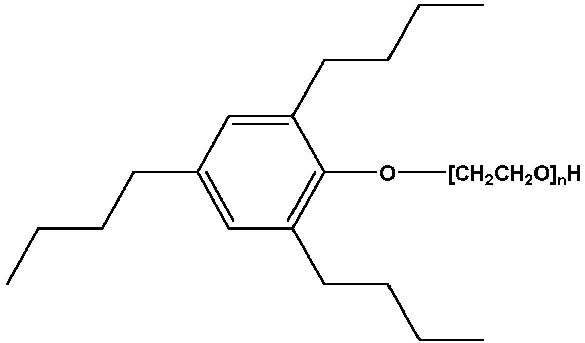 wherein n is selected from 2 to 50
(iv) Anionic alkoxylated tri(n-butyl)phenols of the following structure: 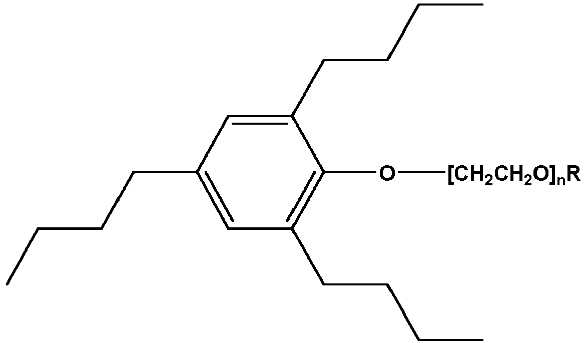 wherein R is selected from SO₃⁻, COO⁻ and PO₃²⁻, preferably selected from SO₃⁻ and COO⁻, wherein n is selected from 6 to 50.

Such compounds are available from industrial suppliers, for example Solvay under the Soprophor trade name, from Clariant under the Emulsogen trade name, Aoki Oil Industrial Co. under the Blaunon trade name, from Stepan under the Makon trade name, and from TOTO Chemical Industry Co. under the Sorpol trade name. Specific examples of suitable compounds are Emulsogen® TS160, Hostapal® BV conc., Sapogenat® T110 or Sapogenat® T139, all from Clariant.

The alkoxylated polyaryl/polyalkyl phenol may be present at levels of 0.5-20wt%, preferably 1-15wt%, most preferably 3-10wt%.

### Additional Enzymes

Preferably the composition of the invention comprises additional enzymes, for example selected from lipases, proteases, nucleases, pectate lyases, cellulases, cutinases, mannanases, galactanases and mixtures thereof. The cleaning compositions preferably comprise one or more additional enzymes from the group selected from nucleases. The cleaning compositions preferably comprises one or more additional enzymes selected from the group amylases, lipases, proteases, pectate lyases, cellulases, cutinases, and mixtures thereof. Preferably, the cleaning compositions comprises one or more additional enzymes selected from amylases and proteases and mixtures thereof. Preferably the cleaning compositions comprise one or more additional enzymes selected from lipases. The compositions may also comprise hemicellulases, peroxidases, xylanases, pectinases, keratinases, reductases, oxidases, phenoloxidases, lipoxygenases, ligninases, pullulanases, tannases, pentosanases, malanases, β-glucanases, arabinosidases, hyaluronidase, chondroitinase, laccase and mixtures thereof. When present in the composition, the aforementioned additional enzymes may be present at levels from about 0.00001% to about 2%, from about 0.0001% to about 1% or even from about 0.001% to about 0.5% enzyme protein by weight of the composition. Preferably the or each additional enzyme is present in the laundering aqueous liquor in an amount of from O.Olppm to 1000 ppm of the active enzyme protein, or from 0.05 or from 0.1ppm to 750 or 500ppm.

### Nucleases

Preferably the composition additionally comprises a nuclease enzyme. The nuclease enzyme is an enzyme capable of cleaving the phosphodiester bonds between the nucleotide subunits of nucleic acids. Suitable nuclease enzymes may be deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof. Nuclease enzymes from class E.C. 3.1.21.x and especially where x=1 are particularly preferred. Nucleases in class E.C. 3.1.22.y cleave at the 5' hydroxyl to liberate 3' phosphomonoesters. Enzymes in class E.C. 3.1.30.z may be preferred as they act on both DNA and RNA and liberate 5'-phosphomonoesters. Suitable examples from class E.C. 3.1.31.2 are described in US2012/0135498A, such as SEQ ID NO:3 therein. Such enzymes are commercially available as DENARASE® enzyme from c-LECTA. Nuclease enzymes from class E.C. 3.1.31.1 produce 3 'phosphomonoesters.

Preferably, the nuclease enzyme comprises a microbial enzyme. The nuclease enzyme may be fungal or bacterial in origin. Bacterial nucleases may be most preferred. Fungal nucleases may be most preferred.

The microbial nuclease is obtainable from *Bacillus,* such as a *Bacillus licheniformis* or *Bacillus subtilis* bacterial nucleases. A preferred nuclease is obtainable from *Bacillus licheniformis*, preferably from strain EI-34-6. A preferred deoxyribonuclease is a variant of *Bacillus licheniformis,* from strain EI-34-6 nucB deoxyribonuclease defined in SEQ ID NO:5 herein, or variant thereof, for example having at least 70% or 75% or 80% or 85% or 90% or 95%, 96%, 97%, 98%, 99% or 100% identical thereto. Other suitable nucleases are defined in SEQ ID NO: 6 herein, or variant thereof, for example having at least 70% or 75% or 80% or 85% or 90% or 95%, 96%, 97%, 98%, 99% or 100% identical thereto. Other suitable nucleases are defined in SEQ ID NO: 7 herein, or variant thereof, for example having at least 70% or 75% or 80% or 85% or 90% or 95%, 96%, 97%, 98%, 99% or 100% identical thereto.

A fungal nuclease is obtainable from *Aspergillus,* for example *Aspergillus oryzae.* A preferred nuclease is obtainable from *Aspergillus oryzae* defined in SEQ ID NO:8 herein, or variant thereof, for example having at least 60% or 70% or75% or 80% or 85% or 90% or 95%, 96%, 97%, 98%, 99% or 100% identical thereto.

Another suitable fungal nuclease is obtainable from *Trichoderma,* for example *Trichoderma harzianum.* A preferred nuclease is obtainable from *Trichoderma harzianum* defined in SEQ ID NO:9 herein, or variant thereof, for example having at least 60% or 70% or75% or 80% or 85% or 90% or 95%, 96%, 97%, 98%, 99% or 100% identical thereto.

Other fungal nucleases include those encoded by the DNA sequences of *Aspergillus oryzae* RIB40, *Aspergillus oryzae* 3.042, *Aspergillus flavus* NRRL3357, *Aspergillus parasiticus* SU-1, *Aspergillus nomius* NRRL13137, *Trichoderma reesei* QM6a, *Trichoderma virens* Gv29-8, *Oidiodendron maius* Zn, *Metarhizium guizhouense* ARSEF 977, *Metarhizium majus* ARSEF *297, Metarhizium robertsii* ARSEF 23, *Metarhizium acridum* CQMa 102, *Metarhizium brunneum* ARSEF 3297, *Metarhizium anisopliae, Colletotrichum fioriniae* PJ7, *Colletotrichum sublineola, Trichoderma atroviride* IMI 206040, *Tolypocladium ophioglossoides* CBS 100239, *Beauveria bassiana* ARSEF 2860, *Colletotrichum higginsianum, Hirsutella minnesotensis* 3608, *Scedosporium apiospermum, Phaeomoniella chlamydospora, Fusarium verticillioides* 7600, *Fusarium oxysporum* f. sp. cubense race 4, *Colletotrichum graminicola* M1.001, *Fusarium oxysporum* FOSC 3-a, *Fusarium avenaceum, Fusarium langsethiae, Grosmannia clavigera* kw1407, *Claviceps purpurea* 20.1, *Verticillium longisporum, Fusarium oxysporum* f. sp. cubense race 1, *Magnaporthe oryzae* 70-15, *Beauveria bassiana* D1-5, *Fusarium pseudograminearum* CS3096, *Neonectria ditissima, Magnaporthiopsis poae* ATCC 64411, *Cordyceps militaris* CM01, *Marssonina brunnea* f. sp. 'multigermtubi' MB_m1, *Diaporthe ampelina, Metarhizium album* ARSEF 1941, *Colletotrichum gloeosporioides* Nara gc5, *Madurella mycetomatis, Metarhizium brunneum* ARSEF 3297, *Verticillium alfalfae* VaMs.102, *Gaeumannomyces graminis* var. tritici R3-111a-1, *Nectria haematococca* mpVI 77-13-4, *Verticillium longisporum, Verticillium dahliae* VdLs.17, *Torrubiella hemipterigena, Verticillium longisporum, Verticillium dahliae* VdLs.17, *Botrytis cinerea* B05.10, *Chaetomium globosum* CBS 148.51, *Metarhizium anisopliae, Stemphylium lycopersici, Sclerotinia borealis* F-4157, *Metarhizium robertsii* ARSEF *23, Myceliophthora thermophila* ATCC 42464, *Phaeosphaeria nodorum* SN15, *Phialophora attae, Ustilaginoidea virens*, *Diplodia seriata, Ophiostoma piceae* UAMH 11346, *Pseudogymnoascus pannorum* VKM F-4515 (FW-2607), *Bipolaris oryzae* ATCC 44560, *Metarhizium guizhouense* ARSEF 977, *Chaetomium thermophilum* var. thermophilum DSM 1495, *Pestalotiopsis fici* W106-1, *Bipolaris zeicola* 26-R-13, *Setosphaeria turcica* Et28A, *Arthroderma otae* CBS 113480 and *Pyrenophora tritici-repentis* Pt-1C-BFP.

Preferably the nuclease is an isolated nuclease.

Preferably the nuclease enzyme is present in the laundering aqueous liquor in an amount of from O.Olppm to 1000 ppm of the nuclease enzyme, or from 0.05 or from 0.1ppm to 750 or 500ppm.

### Acetylglucosaminidases.

Preferably the composition comprises an acetylglucosaminidase enzyme, preferably a β-N-acetylglucosaminidase enzyme from E.C. 3.2.1.52, preferably an enzyme having at least 70%, or at least 75% or at least 80% or at least 85% or at least 90% or at least 95% or at least 96% or at least 97% or at least 98% or at least 99% or at least or 100% identity to SEQ ID NO:10.

### Mannanases

Preferably the composition comprises a mannanase enzyme. The term "mannanase" means a polypeptide having mannan endo-1,4- beta-mannosidase activity (EC 3.2.1.78) from the glycoside hydrolase family 26 that catalyzes the hydrolysis of 1 ,4-3-D-mannosidic linkages in mannans, galactomannans and glucomannans. Alternative names of mannan endo-1,4-beta-mannosidase are 1,4-3-D-mannan mannanohydrolase; endo-1,4-3-mannanase; endo-β-1,4-mannase; β-mannanase B; 3-1,4-mannan 4-mannanohydrolase; endo-3-mannanase; and β-D-mannanase. Preferred mannanases are members of the glycoside hydrolase family 26.

For purposes of the present disclosure, mannanase activity may be determined using the Reducing End Assay as described in the experimental section of WO 2015040159.
Suitable examples from class EC 3.2.1.78 are described in WO 2015040159, such as the mature polypeptide SEQ ID NO: 2 described therein.

Preferred mannanases are variants having at least 60%, at least 65%, at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 11 from *Ascobolus stictoideus*;

Preferred mannanases are variants having at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 12 from *Chaetomium virescens.*

Preferred mannanases are variants having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 13 from *Preussia aemulans.*

Preferred mannanases are variants having at least at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 14 from *Yunnania penicillata.*

Preferred mannanases are variants having at least at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 15 from *Myrothecium roridum.* Preferably the mannanase is an isolated mannanase.

Preferably the mannanase enzyme is present in the cleaning compositions in an amount from 0.001 to 1 wt% based on active protein in the composition, or from 0.005 to 0.5 wt% or from 0.01 to 0.25 wt%. Preferably the mannanase enzyme is present in the laundering aqueous liquor in an amount of from O.Olppm to 1000 ppm of the mannanase enzyme, or from 0.05 or from 0.1ppm to 750 or 500ppm. The compositions of the invention comprising both galactanase and mannanase may be particularly effective against sticky soils and for improved cleaning. It is believed the two enzymes function together in a complementary way.

### Galactanase Enzyme

The endo-beta-1,6-galactanase enzyme is an extracellular polymer-degrading enzyme. The term "endo-beta-1,6-galactanase" or "a polypeptide having endo-beta-1,6-galactanase activity" means a endo-beta-1,6-galactanase activity (EC 3.2.1.164) that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals.

Preferably the galactanase enzyme is selected from Glycoside Hydrolase (GH) Family 30.

Preferably, the endo-beta-1,6-galactanase comprises a microbial enzyme. The endo-beta-1,6-galactanase may be fungal or bacterial in origin. Bacterial endo-beta-1,6-galactanase may be most preferred. Fungal endo-beta-1,6-galactanase may be most preferred.

A bacterial endo-beta-1,6-galactanase is obtainable from Streptomyces, for example Streptomyces davawensis. A preferred endo-beta-1,6-galactanase is obtainable from Streptomyces davawensis JCM 4913 defined in SEQ ID NO: 2 herein, or a variant thereof, for example having at least 40% or 50% or 60% or 70% or 75% or 80% or 85% or 90% or 95%, 96%, 97%, 98%, 99% or 100% identity thereto.

Other bacterial endo-beta-1,6-galactanase include those encoded by the DNA sequences of Streptomyces avermitilis MA-4680 defined in SEQ ID NO: 3 herein, or a variant thereof, for example having at least 40% or 50% or 60% or 70% or 75% or 80% or 85% or 90% or 95%, 96%, 97%, 98%, 99% or 100% identity thereto.

A fungal endo-beta-1,6-galactanase is obtainable from Trichoderma, for example Trichoderma harzianum. A preferred endo-beta-1,6-galactanase is obtainable from Trichoderma harzianum defined in SEQ ID NO: 4 herein, or a variant thereof, for example having at least 40% or 50% or 60% or 70% or 75% or 80% or 85% or 90% or 95%, 96%, 97%, 98%, 99% or 100% identical thereto.

Other fungal endo-beta-1,6-galactanases include those encoded by the DNA sequences of Ceratocystis fimbriata f. sp. Platani, Muscodor strobelii WG-2009a, Oculimacula yallundae, Trichoderma viride GD36A, Thermomyces stellatus, Myceliophthora thermophilia.

Preferably the galactanase has an amino acid sequence having at least 60%, or at least 80%, or at least 90% or at least 95% identity with the amino acid sequence shown in SEQ ID NO:2, SEQ ID NO:3 or SEQ ID NO:4.

Preferably the galactanase is an isolated galactanase.

Preferably the galactanase enzyme is present in a the laundering aqueous solution in an amount of from O.Olppm to 1000 ppm of the galactanase enzyme, or from 0.05 or from 0.1ppm to 750 or 500ppm.

The galactanases may also give rise to biofilm-disrupting effects.

### Glycosyl Hydrolases

The composition may comprise a glycosyl hydrolase selected from GH family 39 and GH family 114 and mixtures thereof, for example as described in co-pending applications having applicants reference numbers CM4645FM and CM4646 FM, respectively.

### Proteases.

Preferably the composition comprises one or more proteases. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
(a) subtilisins (EC 3.4.21.62), especially those derived from *Bacillus,* such as *Bacillus sp., B. lentus, B. alkalophilus, B. subtilis, B. amyloliquefaciens, B. pumilus*, *B. gibsonii,* and *B. akibaii* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569.
(b) trypsin-type or chymotrypsin-type proteases, such as trypsin (*e.g.,* of porcine or bovine origin), including the *Fusarium* protease described in WO 89/06270 and the chymotrypsin proteases derived from *Cellumonas* described in WO 05/052161 and WO 05/052146.
(c) metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* Described in WO2014194032, WO2014194054 and WO2014194117; from *Kribella alluminosa* described in WO2015193488; and from *Streptomyces* and *Lysobacter* described in WO2016075078.
(d) Protease having at least 90% identity to the subtilase from Bacillus sp. TY145, NCIMB 40339, described in WO92/17577 (Novozymes A/S), including the variants of this Bacillus sp TY145 subtilase described in WO2015024739, and WO2016066757.

Especially preferred proteases for the detergent of the invention are polypeptides demonstrating at least 90%, preferably at least 95%, more preferably at least 98%, even more preferably at least 99% and especially 100% identity with the wild-type enzyme from *Bacillus lentus,* comprising mutations in one or more, preferably two or more and more preferably three or more of the following positions, using the BPN' numbering system and amino acid abbreviations as illustrated in WO00/37627, which is incorporated herein by reference:V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W and/or M222S.

Most preferably the protease is selected from the group comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase®, Coronase®, Blaze®, Blaze Ultra® and Esperase® by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (*Bacillus alkalophilus subtilisin* with mutations A230V + S256G + S259N) from Kao.

Especially preferred for use herein in combination with the variant protease of the invention are commercial proteases selected from the group consisting of Properase®, Blaze®, Ultimase®, Everlase®, Savinase®, Excellase®, Blaze Ultra®, BLAP and BLAP variants.

### Lipases

Preferably the composition comprises one or more lipases, including "first cycle lipases" such as those described in U.S. Patent 6,939,702 B1 and US PA 2009/0217464. Preferred lipases are first-wash lipases. In one embodiment of the invention the composition comprises a first wash lipase. First wash lipases includes a lipase which is a polypeptide having an amino acid sequence which: (a) has at least 90% identity with the wild-type lipase derived from *Humicola lanuginosa* strain DSM 4109; (b) compared to said wild-type lipase, comprises a substitution of an electrically neutral or negatively charged amino acid at the surface of the three-dimensional structure within 15A of E1 or Q249 with a positively charged amino acid; and (c) comprises a peptide addition at the C-terminal; and/or (d) comprises a peptide addition at the N-terminal and/or (e) meets the following limitations: i) comprises a negative amino acid in position E210 of said wild-type lipase; ii) comprises a negatively charged amino acid in the region corresponding to positions 90-101 of said wild-type lipase; and iii) comprises a neutral or negative amino acid at a position corresponding to N94 or said wild-type lipase and/or has a negative or neutral net electric charge in the region corresponding to positions 90-101 of said wild-type lipase. Preferred are variants of the wild-type lipase from *Thermomyces lanuginosus* comprising one or more of the T231R and N233R mutations. The wild-type sequence is the 269 amino acids (amino acids 23 - 291) of the Swissprot accession number Swiss-Prot O59952 (derived from *Thermomyces lanuginosus* (*Humicola lanuginosa*))*.* Preferred lipases include those sold under the tradenames Lipex® and Lipolex® and Lipoclean®. Other suitable lipases include those described in European Patent Application No. 12001034.3 or EP2623586.

### Endoglucanases

Other preferred enzymes include microbial-derived endoglucanases exhibiting endo-beta-1,4-glucanase activity (E.C. 3.2.1.4), including a bacterial polypeptide endogenous to a member of the genus Bacillus which has a sequence of at least 90%, 94%, 97% and even 99% identity to the amino acid sequence SEQ ID NO:2 in US7,141,403B2) and mixtures thereof. Suitable endoglucanases are sold under the tradenames Celluclean® and Whitezyme® (Novozymes A/S, Bagsvaerd, Denmark).

### Pectate Lyases

Other preferred enzymes include pectate lyases sold under the tradenames Pectawash®, Pectaway®, Xpect® and mannanases sold under the tradenames Mannaway® (all from Novozymes A/S, Bagsvaerd, Denmark), and Purabrite® (Genencor International Inc., Palo Alto, California).

### Cleaning Cellulase

The cleaning composition described herein may additionally comprise a cleaning cellulase. The cellulase may be an endoglucanase. The cellulase may have endo beta 1,4-glucanase activity and a structure which does not comprise a class A Carbohydrate Binding Module (CBM). A class A CBM is defined according to A. B. Boraston et al. Biochemical Journal 2004, Volume 382 (part 3) pages 769-781. In particular, the cellulase does not comprise a class A CBM from families 1, 2a, 3, 5 and 10.

The cellulase may be a glycosyl hydrolase having enzymatic activity towards amorphous cellulose substrates, wherein the glycosyl hydrolase is selected from GH families 5, 7, 12, 16, 44 or 74. Preferably, the cellulase is a glycosyl hydrolase selected from GH family 5. A preferred cellulase is Celluclean, supplied by Novozymes. This preferred cellulase is described in more detail in WO2002/099091. The glycosyl hydrolase (GH) family definition is described in more detail in Biochem J. 1991, v280, 309-316. Another preferred cellulase is a glycosyl hydrolase having enzymatic activity towards both xyloglucan and amorphous cellulose substrates, wherein the glycosyl hydrolase is selected from GH families 5, 12, 44 or 74. Preferably, the glycosyl hydrolase selected from GH family 44.

For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

Suitable cleaning cellulase glycosyl hydrolases are selected from the group consisting of: GH family 44 glycosyl hydro lases from *Paenibacillus polyxyma* (wild-type) such as XYG1006 described in WO 01/062903 or are variants thereof; GH family 12 glycosyl hydrolases from *Bacillus licheniformis* (wild-type) such as Seq. No. ID: 1 described in WO 99/02663 or are variants thereof; GH family 5 glycosyl hydrolases from *Bacillus agaradhaerens* (wild type) or variants thereof; GH family 5 glycosyl hydrolases from *Paenibacillus* (wild type) such as XYG1034 and XYG 1022described in WO 01/064853 or variants thereof; GH family 74 glycosyl hydrolases from *Jonesia sp.* (wild type) such as XYG1020 described in WO 2002/077242 or variants thereof; and GH family 74 glycosyl hydrolases from *Trichoderma Reesei* (wild type), such as the enzyme described in more detail in Sequence ID no. 2 of WO03/089598, or variants thereof.

Preferred glycosyl hydrolases are selected from the group consisting of: GH family 44 glycosyl hydrolases from *Paenibacillus polyxyma* (wild-type) such as XYG1006 or are variants thereof.

Typically, the cellulase modifies the fabric surface during the laundering process so as to improve the removal of soils adhered to the fabric after the laundering process during wearing and usage of the fabric, in subsequent wash cycles. Preferably, the cellulase modifies the fabric surface during the laundering process so as to improve the removal of soils adhered to the fabric after the laundering process during wearing and usage of the fabric, in the subsequent two, or even three wash cycles.

Typically, the cellulase is used at a concentration of 0.005ppm to 1.0ppm in the aqueous liquor during the first laundering process. Preferably, the cellulase is used at a concentration of 0.02ppm to 0.5ppm in the aqueous liquor during the first laundering process.

### Polymers

The detergent composition may comprise one or more polymers for example for cleaning and/or care. Examples are optionally modified carboxymethylcellulose, poly (ethylene glycol), poly(vinyl alcohol), polycarboxylates such as polyacrylates, maleic/acrylic acid copolymers and lauryl methacrylate/acrylic acid co-polymers and carboxylate polymers.

Suitable carboxylate polymers include maleate/acrylate random copolymer or polyacrylate homopolymer. The carboxylate polymer may be a polyacrylate homopolymer having a molecular weight of from 4,000 Da to 9,000 Da, or from 6,000 Da to 9,000 Da. Other suitable carboxylate polymers are co-polymers of maleic acid and acrylic acid, and may have a molecular weight in the range of from 4,000 Da to 90,000 Da.

Other suitable carboxylate polymers are co-polymers comprising: (i) from 50 to less than 98 wt% structural units derived from one or more monomers comprising carboxyl groups; (ii) from 1 to less than 49 wt% structural units derived from one or more monomers comprising sulfonate moieties; and (iii) from 1 to 49 wt% structural units derived from one or more types of monomers selected from ether bond-containing monomers represented by formulas (I) and (II): 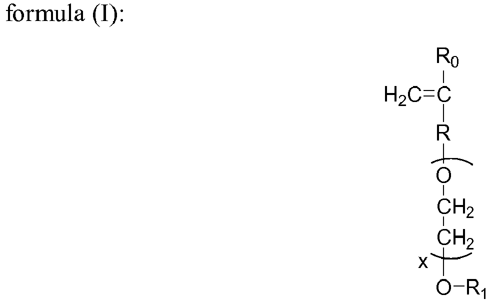 wherein in formula (I), R₀ represents a hydrogen atom or CH₃ group, R represents a CH₂ group, CH₂CH₂ group or single bond, X represents a number 0-5 provided X represents a number 1-5 when R is a single bond, and R₁ is a hydrogen atom or C1 to C20 organic group; 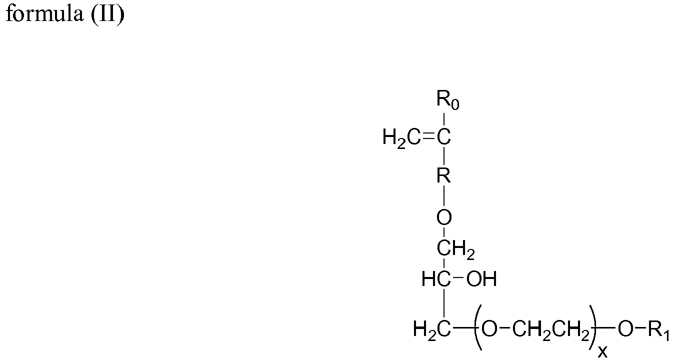 in formula (II), R0 represents a hydrogen atom or CH3 group, R represents a CH2 group, CH2CH2 group or single bond, X represents a number 0-5, and R1 is a hydrogen atom or C1 to C20 organic group. It may be preferred that the polymer has a weight average molecular weight of at least 50kDa, or even at least 70kDa.

The composition may comprise one or more amphiphilic cleaning polymers such as the compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof. In one aspect, this polymer is sulphated or sulphonated to provide a zwitterionic soil suspension polymer.

The composition preferably comprises amphiphilic alkoxylated grease cleaning polymers which have balanced hydrophilic and properties such that they remove grease particles from fabrics and surfaces. Preferred amphiphilic alkoxylated grease cleaning polymers comprise a core structure and a plurality of alkoxylate groups attached to that core structure. These may comprise alkoxylated polyalkylenimines, preferably having an inner polyethylene oxide block and an outer polypropylene oxide block. Typically these may be incorporated into the compositions of the invention in amounts of from 0.005 to 10 wt%, generally from 0.5 to 8 wt%.

Alkoxylated polycarboxylates such as those prepared from polyacrylates are useful herein to provide additional grease removal performance. Such materials are described in WO 91/08281 and PCT 90/01815. Chemically, these materials comprise polyacrylates having one ethoxy side-chain per every 7-8 acrylate units. The side-chains are of the formula -(CH₂CH₂O)ₘ (CH₂)ₙCH₃ wherein m is 2-3 and n is 6-12. The side-chains are ester-linked to the polyacrylate "backbone" to provide a "comb" polymer type structure. The molecular weight can vary, but is typically in the range of about 2000 to about 50,000. Such alkoxylated polycarboxylates can comprise from about 0.05% to about 10%, by weight, of the compositions herein.

Preferably the composition comprises one or more carboxylate polymer, such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da. Typically these are incorporated into the compositions of the invention in amounts from 0.005 to 10 wt%, or from 0.05 to 8 wt%.

The composition preferably comprises a cationically-modified polysaccharide polymer. Preferably, the cationic polysaccharide polymer is selected from cationically modified hydroxyethyl cellulose, cationically modified hydroxypropyl cellulose, cationically and hydrophobically modified hydroxyethyl cellulose, cationically and hydrophobically modified hydroxypropyl cellulose, or a mixture thereof, more preferably cationically modified hydroxyethyl cellulose, cationically and hydrophobically modified hydroxyethyl cellulose, or a mixture thereof.

Soil release polymer: The composition may comprise a soil release polymer. A suitable soil release polymer has a structure as defined by one of the following structures (I), (II) or (III):

(I) -[(OCHR¹-CHR²)ₐ-O-OC-Ar-CO-]_{d}

(II) -[(OCHR³-CHR)_{b}-O-OC-sAr-CO-]ₑ

(III) -[(OCHR⁵-CHR⁶)_{c}-OR⁷]_{f}

wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or mixtures thereof;
R¹, R², R³, R⁴, R⁵ and R⁶ are independently selected from H or C₁-C₁₈ n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group.

Suitable soil release polymers are sold by Clariant under the TexCare® series of polymers, e.g. TexCare® SRA100, SRA300, SRN100, SRN170, SRN240, SRN260, SRN300 and SRN325 and TexCare® SRA300, SRN240 and SRA 300 being particularly preferred. Other suitable soil release polymers are sold by Solvay under the Repel-o-Tex® series of polymers, e.g. Repel-o-Tex® SF2, SRP6 and Repel-o-Tex® Crystal. Preferably the composition comprises one or more soil release polymers. Other suitable soil release polymers are Marloquest polymers, such as Marloquest SL supplied by Sasol.

Anti-redeposition polymer: Suitable anti-redeposition polymers include polyethylene glycol polymers and/or polyethyleneimine polymers.

Suitable polyethylene glycol polymers include random graft co-polymers comprising: (i) hydrophilic backbone comprising polyethylene glycol; and (ii) hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1:1 to 1:5, or from 1:1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22. Suitable polyethylene glycol polymers are described in WO08/007320.

Typically these polymers when present are each incorporated into the compositions of the invention in amounts from 0.005 to 10 wt%, more usually from 0.05 to 8 wt%.

Cellulosic polymer: Preferably the composition comprises a cellulosic polymer. Suitable cellulosic polymers are selected from alkyl cellulose, alkyl alkoxyalkyl cellulose, carboxyalkyl cellulose, alkyl carboxyalkyl cellulose, sulphoalkyl cellulose, more preferably selected from carboxymethyl cellulose, methyl cellulose, methyl hydroxyethyl cellulose, methyl carboxymethyl cellulose, and mixures thereof.

Preferred carboxymethyl celluloses have a degree of carboxymethyl substitution from 0.5 to 0.9 and a molecular weight from 100,000 Da to 300,000 Da.
Suitable carboxymethyl celluloses have a degree of substitution greater than 0.65 and a degree of blockiness greater than 0.45, e.g. as described in WO09/154933.

Care polymers: Suitable care polymers include cellulosic polymers that are cationically modified and/or hydrophobically modified. Such modified cellulosic polymers can provide anti-abrasion benefits and dye lock benefits to fabric during the laundering cycle. Suitable cellulosic polymers include cationically modified hydroxyethyl cellulose. Suitable care polymers also include guar polymers that are cationically and/or hydrophobically modified. Other suitable care polymers include dye lock polymers, for example the condensation oligomer produced by the condensation of imidazole and epichlorhydrin, preferably in ratio of 1:4:1. A suitable commercially available dye lock polymer is Polyquart® FDI (Cognis).

Other suitable care polymers include amino-silicone, which can provide fabric feel benefits and fabric shape retention benefits.

Alkoxylated polyalkyleneimine: The composition may comprise an alkoxylated polyalkyleneimine, wherein said alkoxylated polyalkyleneimine has a polyalkyleneimine core with one or more side chains bonded to at least one nitrogen atom in the polyalkyleneimine core, wherein said alkoxylated polyalkyleneimine has an empirical formula (I) of (PEI)ₐ-(EO)_{b}-R₁, wherein a is the average number-average molecular weight (MW_{PEI}) of the polyalkyleneimine core of the alkoxylated polyalkyleneimine and is in the range of from 100 to 100,000 Daltons, wherein b is the average degree of ethoxylation in said one or more side chains of the alkoxylated polyalkyleneimine and is in the range of from 5 to 40, and wherein R₁ is independently selected from the group consisting of hydrogen, C₁-C₄ alkyls, and combinations thereof.

The composition may comprise an alkoxylated polyalkyleneimine, wherein said alkoxylated polyalkyleneimine has a polyalkyleneimine core with one or more side chains bonded to at least one nitrogen atom in the polyalkyleneimine core, wherein the alkoxylated polyalkyleneimine has an empirical formula (II) of (PEI)ₒ-(EO)ₘ(PO)ₙ-R₂ or (PEI)ₒ-(PO)ₙ(EO)ₘ-R₂, wherein o is the average number-average molecular weight (MW_{PEI}) of the polyalkyleneimine core of the alkoxylated polyalkyleneimine and is in the range of from 100 to 100,000 Daltons, wherein m is the average degree of ethoxylation in said one or more side chains of the alkoxylated polyalkyleneimine which ranges from 10 to 50, wherein n is the average degree of propoxylation in said one or more side chains of the alkoxylated polyalkyleneimine which ranges from 1 to 50, and wherein R₂ is independently selected from the group consisting of hydrogen, C₁-C₄ alkyls, and combinations thereof.

### Dye control agent

The cleaning composition may comprise a dye control agent typically present in the composition at a level of from about 0.02% to about 1%, or from about 0.05% to about 0.5%, by weight of the cleaning composition.

The dye control agent may be selected from the group consisting of: (i) a sulfonated phenol / formaldehyde polymer; (ii) a urea derivative; (iii) polymers of ethylenically unsaturated monomers, where the polymers are molecularly imprinted with dye; (iv) fibers consisting of water-insoluble polyamide, wherein the fibers have an average diameter of not more than about 2 µm; (v) a polymer obtainable from polymerizing benzoxazine monomer compounds; and (vi) combinations thereof. These dye control agents are described in more detail below.

### (i) Sulfonated phenol / formaldehyde polymer

The dye control agent may comprise a sulfonated phenol / formaldehyde polymer. The sulfonated phenol / formaldehyde polymer may be selected from the product of the condensation of formaldehyde with phenol, cresols, xylenols, nonyl phenol, octyl phenol, butylphenol, phenylphenol, 2,2-bis-4-hydroxyphenylpropane, anisole, resorcinol, bisphenol A, 4,4'-, 2,2'- or 4,2'-dihydroxydiphenyl ether, phenolsulfonic acid, anisole sulfonic acid, dioxydiphenylsulfone, 4-hydroxydiphenylsulfone, naphthol or naphtholsulfonic acid. Suitable examples include Suparex® O.IN (M1), Nylofixan® P (M2), Nylofixan® PM (M3), and Nylofixan® HF (M4) (all supplied by Archroma, Reinach, Switzerland).

### (ii) Urea derivative

The dye control agent may comprise a urea derivative. The urea derivative may have a structure according to Formula I,

Formula I (A)ₖAr-NH-C(O)-NH-Ar(A)₁-NH[-C(O)-NH-L-NH-C(O)-NH-Ar(A)ₘ NH]ₙ-C(O)-NH-Ar(A)ₖ

in which
Ar denotes an aromatic group, a stilbene group, or a linear, branched, or cyclic, saturated or once or several times ethylenically unsaturated hydrocarbon group with 1 to 12 carbon atoms;
L denotes an arylene or stilbene group;
A denotes -SO₃M or -CO₂M;
M denotes H or an alkali metal atom;
k and m irrespective of each other denote 0, 1, 2 or 3, and l+m≧1;
n denotes a number of from 1 to 6.

Suitable examples of urea derivatives include compounds according to Formulas II and III, below.

Formula II is below, in which Ph is a phenyl group, n is 1, 2, 3 or 4, the substituents - SO₃H are in ortho position, and the substituents -CH₃ is in ortho position:

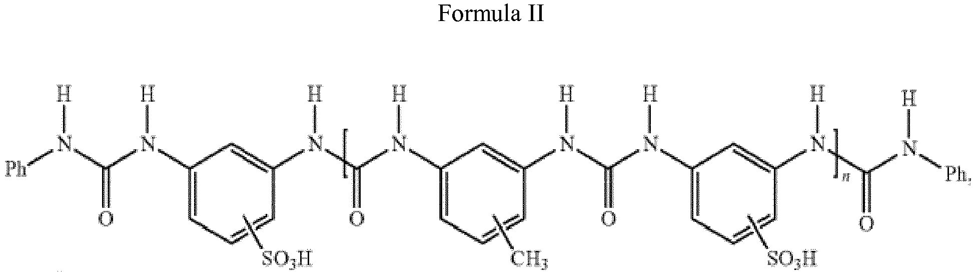

Formula III is below, in which Ph is a phenyl group, n is 1, 2, 3, or 4, the substituents-SO₃H are in ortho positions, and the substituent -CH₃ is in ortho position:

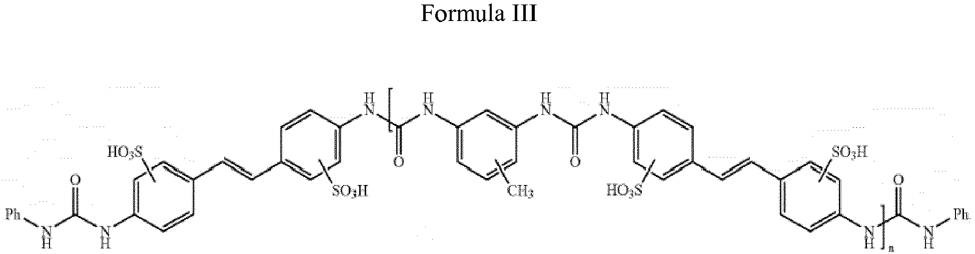

### (iii) Polymers of ethylenically unsaturated monomers, where the polymers are molecularly imprinted with dye

The dye control agent may comprise polymers of ethylenically unsaturated monomers, where the polymers are molecularly imprinted with dye. Methods of producing the molecular imprinted dyes are given in G. Z. Kyzas et al, Chemical Engineering Journal, Volume 149, Issues 1-3, 1 July 2009, Pages 263-272.

One illustrative polymer can be synthesized as follows: 4.05 g (20 mmol) of ethylene glycol monomethacrylate, 0.34 g (4 mmol) of methacrylic acid, and 2.18 g (3.2 mmol) of disodium-8-anilino-5-[[4-[(3-sulfonatophenyl)diazenyl]naphthalen-1-yl]diazenyl]naphthalene-1-sulfonate (Acid Blue 113) in 100 mL of dimethylformamide are charged under a nitrogen atmosphere and stirred for 2 hours at room temperature. Next, 22 mg of azoisobutyronitrile is added; the reaction mixture is degassed for 15 minutes in the ultrasonic bath and is stirred for 12 hours at 75° C. The residue is separated, is washed with acetone and hot water, and is extracted with 500 mL of methanol in a Soxhlet extractor for 8 hours. After drying, 3.5 g of a brittle, dark purple product is obtained.

### (iv) Fibers consisting of water-insoluble polyamide

The dye control agent may comprise fibers consisting of water-insoluble polyamide. The average diameter of the fibers may be not more than 2 µm. Exemplary water-insoluble polyamides fibers include those produced from polyamide-6 and/or polyamide 6,6. The average fiber diameter can be measured by Scanning Electron Microscopy in conjunction with suitable image analysis software, for example the FiberMetric® fiber measurement software supplied by Phenom-World B.V., Eindhoven, The Netherlands.

### (v) Polymer obtainable from polymerizing benzoxazine monomer compounds

The dye control agent may comprise a polymer obtainable from polymerizing benzoxazine monomer compounds. The polymer obtainable from polymerizing benzoxazine monomer compounds may be selected from Formula IV, Formula V, or mixtures thereof: 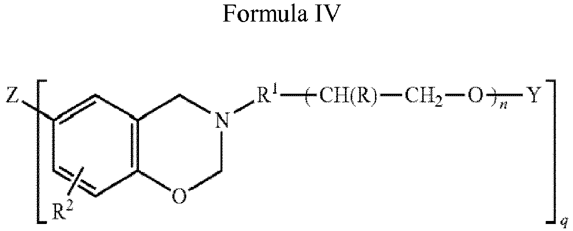 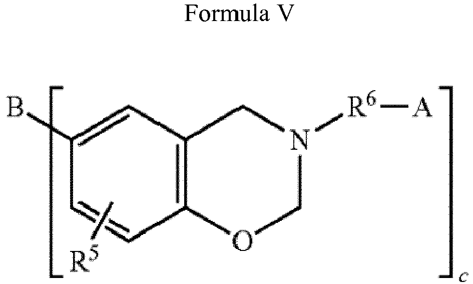 wherein for Formula IV and Formula V:
q is a whole number from 1 to 4,
n is a number from 2 to 20 000,
R in each repeat unit is selected independently of each other from hydrogen or linear or branched, optionally substituted alkyl groups that comprise 1 to 8 carbon atoms,
Z is selected from hydrogen (for q=1), alkyl (for q=1), alkylene (for q=2 to 4), carbonyl (for q=2), oxygen (for q=2), sulfur (for q=2), sulfoxide (for q=2), sulfone (for q=2) and a direct, covalent bond (for q=2),
R¹ stands for a covalent bond or a divalent linking group that contains 1 to 100 carbon atoms,
R² is selected from hydrogen, halogen, alkyl, alkenyl, and/or a divalent group that makes a corresponding naphthoxazine structure from the benzoxazine structure,
Y is selected from linear or branched, optionally substituted, alkyl groups that contain 1 to 15 carbon atoms, cycloaliphatic groups that optionally comprise one or more heteroatoms, aryl groups that optionally comprise one or more heteroatoms, and -(C=O)R₃, wherein R₃ is selected from linear or branched, optionally substituted, alkyl groups containing 1 to 15 carbon atoms and X-R₄, wherein X is selected from S, O, and NH and R₄ is selected from linear or branched, optionally substituted, alkyl groups containing 1 to 15 carbon atoms,
c is a whole number from 1 to 4,
B is selected from hydrogen (for c=1), alkyl (for c=1), alkylene (for c=2 to 4), carbonyl (for c=2), oxygen (for c=2), sulfur (for c=2), sulfoxide (for c=2), sulfone (for c=2) and a direct, covalent bond (for c=2), A is a hydroxyl group or a nitrogen-containing heterocycle,
R⁵ is selected from hydrogen, halogen, alkyl and alkenyl, or R⁵ is a divalent group that makes a corresponding naphthoxazine structure from the benzoxazine structure, and
R⁶ stands for a covalent bond or is a divalent linking group that contains 1 to 100 carbon atoms.

The polymer obtainable from polymerizing benzoxazine monomer compounds may be a compound according to Formula VI: 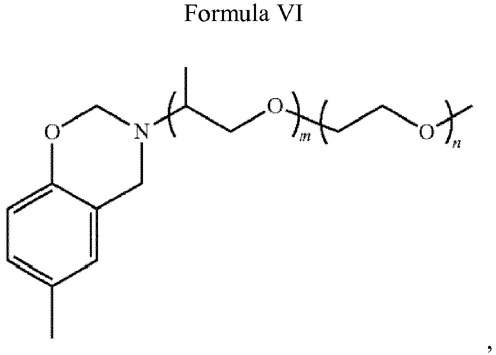 wherein typically, m = 35, and wherein n = 6.

The compound according to Formula VI may be produced by adding a solution of 16.22 p-cresol in 50ml ethyl acetate dropwise over a period of 10 minutes to a solution of 9.38g paraformaldehyde (96% conc.) in 50ml ethyl acetate. 309.9g Jeffamin M2070 (Huntsman, EO/PO ratio 10:31) in 200ml ethyl acetate was then added over a period of 30 minutes, the temperature being maintained below 10°C. After stirring for 10 minutes, the reaction mixture was heated under reflux for 6 h. After cooling, the reaction mixture was filtered and the solvent together with any formed water were removed under vacuum. 318.90g of the corresponding polymerisable benzoxazine compound was obtained.

### Amines

The cleaning compositions described herein may contain an amine. The cleaning compositions may include from about 0.1% to about 10%, or from about 0.2% to about 5%, or from about 0.5% to about 4%, or from about 0.1% to about 4%, or from about 0.1% to about 2%, by weight of the composition, of an amine. The amine can be subjected to protonation depending on the pH of the cleaning medium in which it is used. Non-limiting examples of amines include, but are not limited to, etheramines, cyclic amines, polyamines, oligoamines (e.g., triamines, diamines, pentamines, tetraamines), or combinations thereof. The compositions described herein may comprise an amine selected from the group consisting of oligoamines, etheramines, cyclic amines, and combinations thereof. In some aspects, the amine is not an alkanolamine. In some aspects, the amine is not a polyalkyleneimine. Examples of suitable oligoamines include tetraethylenepentamine, triethylenetetraamine, diethylenetriamine, and mixtures thereof. Etheramines and cyclic amines may be particularly preferred.

Bleach: Suitable bleach includes sources of hydrogen peroxide, bleach activators, bleach catalysts, pre-formed peracids and any combination thereof. A particularly suitable bleach includes a combination of a source of hydrogen peroxide with a bleach activator and/or a bleach catalyst.

Source of hydrogen peroxide: Suitable sources of hydrogen peroxide include sodium perborate and/or sodium percarbonate.

Bleach activator: Suitable bleach activators include tetra acetyl ethylene diamine and/or alkyl oxybenzene sulphonate.

Bleach catalyst: The composition may comprise a bleach catalyst. Suitable bleach catalysts include oxaziridinium bleach catalysts, transistion metal bleach catalysts, especially manganese and iron bleach catalysts. A suitable bleach catalyst has a structure corresponding to general formula below: 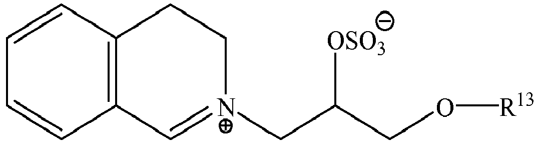 wherein R¹³ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, iso-nonyl, isodecyl, iso-tridecyl and iso-pentadecyl.

Pre-formed peracid: Suitable pre-form peracids include phthalimido-peroxycaproic acid. However, it is preferred that the composition is substantially free of pre-formed peracid. By: "substantially free" it is meant: "no deliberately added".

Other enzymes: Other suitable enzymes are bleaching enzymes, such as peroxidases/oxidases, which include those of plant, bacterial or fungal origin and variants thereof. Commercially available peroxidases include Guardzyme® (Novozymes A/S). Other suitable enzymes include choline oxidases and perhydrolases such as those used in Gentle Power Bleach™.

Brightener: Suitable fluorescent brighteners include: di-styryl biphenyl compounds, e.g. Tinopal® CBS-X, di-amino stilbene di-sulfonic acid compounds, e.g. Tinopal® DMS pure Xtra and Blankophor® HRH, and Pyrazoline compounds, e.g. Blankophor® SN, and coumarin compounds, e.g. Tinopal® SWN.

Preferred brighteners are: sodium 2 (4-styryl-3 -sulfophenyl)-2H-napthol[1,2-d]triazole, disodium 4,4'-bis{[(4-anilino-6-(N methyl-N-2 hydroxyethyl)amino 1,3,5-triazin-2-yl)]amino}stilbene-2-2' disulfonate, disodium 4,4'-bis{[(4-anilino-6-morpholino-1,3,5-triazin-2-yl)]amino}stilbene-2-2' disulfonate, and disodium 4,4'- bis(2-sulfostyryl)biphenyl. A suitable fluorescent brightener is C.I. Fluorescent Brightener 260, which may be used in its beta or alpha crystalline forms, or a mixture of these forms.

### pH

Preferably the pH of the compositions of the invention in a 1 wt% solution of deionised water is from above 6.5 to 11, more preferably from 7 to below 9. Should the compositions be used for antibacterial purposes, an acidic pH, typically from 1 to 6.5, preferably from 1 to 3 may be useful. Preferred cleaning compositions according to the invention, especially laundry cleaning compositions provide a pH less than 9, preferably from 7 to 8.9 for a 1 wt% solution in deionised water.

### Encapsulated Benefit Agent

The composition may further comprise an encapsulated benefit agent. The encapsulated benefit may comprise a shell surrounding a core. The core may comprise a benefit agent. The benefit agent may comprise perfume raw materials.

The shell may comprise a material selected from the group consisting of aminoplast copolymer, an acrylic, an acrylate, and mixtures thereof. The aminoplast copolymer may be melamine-formaldehyde, urea-formaldehyde, cross-linked melamine formaldehyde, or mixtures thereof.

The shell may be coated with one or more materials, such as a polymer, that aids in the deposition and/or retention of the perfume microcapsule on the site that is treated with the composition disclosed herein. The polymer may be a cationic polymer selected from the group consisting of polysaccharides, cationically modified starch, cationically modified guar, polysiloxanes, poly diallyl dimethyl ammonium halides, copolymers of poly diallyl dimethyl ammonium chloride and vinyl pyrrolidone, acrylamides, imidazoles, imidazolinium halides, imidazolium halides, poly vinyl amine, copolymers of poly vinyl amine and N-vinyl formamide, and mixtures thereof.

The core may comprise a benefit agent. Suitable benefit agents include a material selected from the group consisting of perfume raw materials, silicone oils, waxes, hydrocarbons, higher fatty acids, essential oils, lipids, skin coolants, vitamins, sunscreens, antioxidants, glycerine, catalysts, bleach particles, silicon dioxide particles, malodor reducing agents, odor-controlling materials, chelating agents, antistatic agents, softening agents, insect and moth repelling agents, colorants, antioxidants, chelants, bodying agents, drape and form control agents, smoothness agents, wrinkle control agents, sanitization agents, disinfecting agents, germ control agents, mold control agents, mildew control agents, antiviral agents, drying agents, stain resistance agents, soil release agents, fabric refreshing agents and freshness extending agents, chlorine bleach odor control agents, dye fixatives, dye transfer inhibitors, color maintenance agents, optical brighteners, color restoration/rejuvenation agents, anti-fading agents, whiteness enhancers, anti-abrasion agents, wear resistance agents, fabric integrity agents, anti-wear agents, anti-pilling agents, defoamers, anti-foaming agents, UV protection agents, sun fade inhibitors, anti-allergenic agents, enzymes, water proofing agents, fabric comfort agents, shrinkage resistance agents, stretch resistance agents, stretch recovery agents, skin care agents, glycerin, and natural actives, antibacterial actives, antiperspirant actives, cationic polymers, dyes and mixtures thereof. The benefit agent may comprise perfume raw materials.

The composition may comprise, based on total composition weight, from about 0.01% to about 10%, or from about 0.1% to about 5%, or from about 0.2% to about 1%, of encapsulated benefit agent. The encapsulated benefit agent may be friable and/or have a mean particle size of from about 10 microns to about 500 microns or from about 20 microns to about 200 microns.

Suitable encapsulated benefit agents may be obtained from Encapsys, LLC, of Appleton, Wisconsin USA.

Formaldehyde scavengers may also be used in or with such encapsulated benefit agents.

### Methods of Making the Composition

The present disclosure relates to methods of making the compositions described herein. The compositions of the invention may be solid (for example granules or tablets) or liquid form. Preferably the compositions are in liquid form. They may be made by any process chosen by the formulator, including by a batch process, a continuous loop process, or combinations thereof.

When in the form of a liquid, the compositions of the invention may be aqueous (typically above 2 wt% or even above 5 or 10 wt% total water, up to 90 or up to 80wt% or 70 wt% total water) or non-aqueous (typically below 2 wt% total water content). Typically the compositions of the invention will be in the form of an aqueous solution or uniform dispersion or suspension of optical brightener, DTI and optional additional adjunct materials, some of which may normally be in solid form, that have been combined with the normally liquid components of the composition, such as the liquid alcohol ethoxylate nonionic, the aqueous liquid carrier, and any other normally liquid optional ingredients. Such a solution, dispersion or suspension will be acceptably phase stable. When in the form of a liquid, the detergents of the invention preferably have viscosity from 1 to 1500 centipoises (1-1500 mPa*s), more preferably from 100 to 1000 centipoises (100-1000 mPa*s), and most preferably from 200 to 500 centipoises (200-500 mPa*s) at 20s-1 and 21°C. Viscosity can be determined by conventional methods. Viscosity may be measured using an AR 550 rheometer from TA instruments using a plate steel spindle at 40 mm diameter and a gap size of 500 µm. The high shear viscosity at 20s-1 and low shear viscosity at 0.05-1 can be obtained from a logarithmic shear rate sweep from 0.1-1 to 25-1 in 3 minutes time at 21C. The preferred rheology described therein may be achieved using internal existing structuring with detergent ingredients or by employing an external rheology modifier. More preferably the detergents, such as detergent liquid compositions have a high shear rate viscosity of from about 100 centipoise to 1500 centipoise, more preferably from 100 to 1000 cps. Unit Dose detergents, such as detergent liquid compositions have high shear rate viscosity of from 400 to 1000cps. Detergents such as laundry softening compositions typically have high shear rate viscosity of from 10 to 1000, more preferably from 10 to 800 cps, most preferably from 10 to 500 cps. Hand dishwashing compositions have high shear rate viscosity of from 300 to 4000 cps, more preferably 300 to 1000 cps.

The cleaning and/or treatment compositions in the form of a liquid herein can be prepared by combining the components thereof in any convenient order and by mixing, e.g., agitating, the resulting component combination to form a phase stable liquid detergent composition. In a process for preparing such compositions, a liquid matrix is formed containing at least a major proportion, or even substantially all, of the liquid components, e.g., nonionic surfactant, the non-surface active liquid carriers and other optional liquid components, with the liquid components being thoroughly admixed by imparting shear agitation to this liquid combination. For example, rapid stirring with a mechanical stirrer may usefully be employed. While shear agitation is maintained, substantially all of any anionic surfactants and the solid form ingredients can be added. Agitation of the mixture is continued, and if necessary, can be increased at this point to form a solution or a uniform dispersion of insoluble solid phase particulates within the liquid phase. After some or all of the solid-form materials have been added to this agitated mixture, particles of any enzyme material to be included, e.g., enzyme granulates, are incorporated. As a variation of the composition preparation procedure hereinbefore described, one or more of the solid components may be added to the agitated mixture as a solution or slurry of particles premixed with a minor portion of one or more of the liquid components. After addition of all of the composition components, agitation of the mixture is continued for a period of time sufficient to form compositions having the requisite viscosity and phase stability characteristics. Frequently this will involve agitation for a period of from about 30 to 60 minutes.

The adjunct ingredients in the compositions of this invention may be incorporated into the composition as the product of the synthesis generating such components, either with or without an intermediate purification step. Where there is no purification step, commonly the mixture used will comprise the desired component or mixtures thereof (and percentages given herein relate to the weight percent of the component itself unless otherwise specified) and in addition unreacted starting materials and impurities formed from side reactions and/or incomplete reaction. For example, for an ethoxylated or substituted component, the mixture will likely comprise different degrees of ethoxylation/substitution.

### Method of Use

The present disclosure relates to methods of using the cleaning compositions of the present disclosure to clean a surface, such as a hard surface or textile. In general, the method includes mixing the cleaning composition as described herein with water to form an aqueous liquor and contacting a surface, preferably a textile, with the aqueous liquor in a washing step. The target surface may include a greasy soil.

The compositions of this invention, typically prepared as hereinbefore described, can be used to form aqueous washing/treatment solutions for use in the laundering/treatment of fabrics and/or hard surfaces. Generally, an effective amount of such a composition is added to water, for example in a conventional automatic washing machine, to form such aqueous cleaning/washing solutions. The aqueous washing solution so formed is then contacted, typically under agitation, with the hard surfaces or fabrics to be laundered/treated therewith. An effective amount of the detergent composition herein added to water to form aqueous cleaning solutions can comprise amounts sufficient to form from about 500 to 25,000 ppm, or from 500 to 15,000 ppm of composition in aqueous washing solution, or from about 1,000 to 3,000 ppm of the detergent compositions herein will be provided in aqueous washing solution.

Typically, the wash liquor is formed by contacting the cleaning composition with wash water in such an amount so that the concentration of the detergent in the wash liquor is from above 0g/l to 5g/l, or from lg/1, and to 4.5g/l, or to 4.0g/l, or to 3.5g/l, or to 3.0g/l, or to 2.5g/l, or even to 2.0g/l, or even to 1.5g/l. The method of cleaning fabric or textile may be carried out in a top-loading or front-loading automatic washing machine, or can be used in a hand-wash application. In these applications, the wash liquor formed and concentration of cleaning composition in the wash liquor is that of the main wash cycle. Any input of water during any optional rinsing step(s) is not included when determining the volume of the wash liquor.

The wash liquor may comprise 40 litres or less of water, or 30 litres or less, or 20 litres or less, or 10 litres or less, or 8 litres or less, or even 6 litres or less of water. The wash liquor may comprise from above 0 to 15 litres, or from 2 litres, and to 12 litres, or even to 8 litres of water. Typically from 0.01kg to 2kg of fabric per litre of wash liquor is dosed into said wash liquor. Typically from 0.01kg, or from 0.05kg, or from 0.07kg, or from 0.10kg, or from 0.15kg, or from 0.20kg, or from 0.25kg fabric per litre of wash liquor is dosed into said wash liquor. Optionally, 50g or less, or 45g or less, or 40g or less, or 35g or less, or 30g or less, or 25g or less, or 20g or less, or even 15g or less, or even 10g or less of the composition is contacted to water to form the wash liquor. Such compositions are typically employed at concentrations of from about 500 ppm to about 15,000 ppm in solution. The temperature of the wash liquor typically ranges from about 5 °C to about 90 °C, preferably from 5 °C to about 40 °C, more preferably from 5 °C to about 30 °C, or even from 5 °C to about 20 °C. When the surface being cleaned comprises a fabric, the water to fabric ratio is typically from about 1:1 to about 30:1. Typically the wash liquor comprising the detergent of the invention has a pH of from 3 to 11.5, more preferably from 7.5 to 12.

In one aspect, such method comprises the steps of optionally washing and/or rinsing said surface or fabric, contacting said surface or fabric with any composition disclosed in this specification then optionally washing and/or rinsing said surface or fabric is disclosed, with an optional drying step.

Drying of such surfaces or fabrics may be accomplished by any one of the common means employed either in domestic or industrial settings: machine drying or open-air drying. The fabric may comprise any fabric capable of being laundered in normal consumer or institutional use conditions, and the invention is particularly suitable for synthetic textiles such as polyester and nylon and especially for treatment of mixed fabrics and/or fibres comprising synthetic and cellulosic fabrics and/or fibres. As examples of synthetic fabrics are polyester, nylon, these may be present in mixtures with cellulosic fibres, for example, polycotton fabrics. The solution typically has a pH of from 7 to 11, more usually 8 to 10.5. The compositions are typically employed at concentrations from 500 ppm to 5,000 ppm in solution. The water temperatures typically range from about 5 °C to about 90 °C. The water to fabric ratio is typically from about 1:1 to about 30:1.

### EXAMPLES

### EXAMPLE

The wash performance was determined using a 6 well plate (Costar 3516). Wash solution was prepared by adding 1.87g/L dosage of soluble unit dose detergent, detergent was dissolved during magnet stirring for 2 min. 5 ml of this solution was used for the wash step. Several washes were carried out with varying addition of enzymes, as set out in the table below. Wash temperature was set at 20°C & agitation at 450rpm for 30 min, followed by a 2 min rinse step. A single 2.5 x 2.5cm starch stain swatch was added per well, Tapioca (CS-29) & Salad dressing (CS-06) stains sources from WFK were used. 4 repeats were done per condition. The stains were analyzed using image analysis and results are presented as stain removal index (SRI) values, where 0 represents no removal and 100 complete removal.

| Tapioca starch | SRI / 30min wash/20°C |
|---|---|
| Nil enzyme | 19.0 |
| Isoamylase 0.65ppm | 14.8 |
| Amylase 0.65ppm | 44.1 |
| Amylase 1.3ppm | 51.4 |
| Amylase 0.65ppm + Isoamylase 0.65ppm | 54.9 |

| Salad dressing | SRI / 30min wash/20°C |
|---|---|
| Nil enzyme | 5.2 |
| Isoamylase 0.65ppm | 7.4 |
| Amylase 0.65ppm | 6.1 |
| Amylase 1.3ppm | 6.0 |
| Amylase 0.65ppm + Isoamylase 0.65ppm | 8.8 |

### FORMULATION EXAMPLES

The following are illustrative examples of cleaning compositions according to the present disclosure and are not intended to be limiting.

### Examples 1-7: Heavy Duty Liquid laundry detergent compositions.

| **Ingredients** | **1** | **2** | **3** | **4** | **5** | **6** | **7** |
|---|---|---|---|---|---|---|---|
| | % weight | | | | | | |
| AE_{1.8}S | 6.77 | 2.16 | 1.36 | 1.30 | - | - | - |
| AE₃S | - | 3.0 | - | - | 0.45 | - | - |
| LAS | 0.86 | 2.06 | 2.72 | 0.68 | 0.95 | 1.56 | 3.55 |
| HSAS | 1.85 | 2.63 | 1.02 | - | - | - | - |
| AE9 | 6.32 | 9.85 | 10.20 | 7.92 | | | |
| AE8 | | | | | | | 35.45 |
| AE7 | | | | | 8.40 | 12.44 | |
| C₁₂₋₁₄ dimethyl Amine Oxide | 0.30 | 0.73 | 0.23 | 0.37 | - | - | - |
| C₁₂₋₁₈ Fatty Acid | 0.80 | 1.90 | 0.60 | 0.99 | 1.20 | - | 15.00 |
| Citric Acid | 2.50 | 3.96 | 1.88 | 1.98 | 0.90 | 2.50 | 0.60 |
| Optical Brightener 1 | 1.00 | 0.80 | 0.10 | 0.30 | 0.05 | 0.50 | 0.001 |
| Optical Brightener 3 | 0.001 | 0.05 | 0.01 | 0.20 | 0.50 | - | 1.00 |
| Sodium formate | 1.60 | 0.09 | 1.20 | 0.04 | 1.60 | 1.20 | 0.20 |
| DTI 1 | 0.32 | 0.05 | - | 0.60 | 0.10 | 0.60 | 0.01 |
| DTI 2 | 0.32 | 0.10 | 0.60 | 0.60 | 0.05 | 0.40 | 0.20 |
| Sodium hydroxide | 2.30 | 3.80 | 1.70 | 1.90 | 1.70 | 2.50 | 2.30 |
| Monoethano lamine | 1.40 | 1.49 | 1.00 | 0.70 | - | - | - |
| Diethylene glycol | 5.50 | - | 4.10 | - | - | - | - |
| Chelant 1 | 0.15 | 0.15 | 0.11 | 0.07 | 0.50 | 0.11 | 0.80 |
| 4-formyl-phenylboronic acid | - | - | - | - | 0.05 | 0.02 | 0.01 |
| Sodium tetraborate | 1.43 | 1.50 | 1.10 | 0.75 | - | 1.07 | - |
| Ethanol | 1.54 | 1.77 | 1.15 | 0.89 | - | 3.00 | 7.00 |
| Polymer 1 | 0.10 | - | - | - | - | - | 2.00 |
| Polymer 2 | 0.30 | 0.33 | 0.23 | 0.17 | - | - | - |
| Polymer 3 | - | - | - | - | - | - | 0.80 |
| Polymer 4 | 0.80 | 0.81 | 0.60 | 0.40 | 1.00 | 1.00 | - |
| 1,2-Propanediol | - | 6.60 | - | 3.30 | 0.50 | 2.00 | 8.00 |
| Structurant | 0.10 | - | - | - | - | - | 0.10 |
| Perfume | 1.60 | 1.10 | 1.00 | 0.80 | 0.90 | 1.50 | 1.60 |
| Perfume encapsulate | 0.10 | 0.05 | 0.01 | 0.02 | 0.10 | 0.05 | 0.10 |
| Protease | 0.80 | 0.60 | 0.70 | 0.90 | 0.70 | 0.60 | 1.50 |
| Mannanase | 0.07 | 0.05 | 0.045 | 0.06 | 0.04 | 0.045 | 0.10 |
| Amylase 1 | - | - | 0.30 | 0.10 | - | - | 0.10 |
| Amylase 2 | - | 0.10 | 0.10 | - | 0.07 | - | - |
| Amylase 4, preferably from c) and/or h) | 0.30 | 0.1 | - | 0.15 | 0.03 | 0.40 | 0.10 |
| Isoamylase | 0.30 | 0.20 | 0.10 | 0.07 | 0.2 | 0.02 | 0.30 |
| Xyloglucanase | 0.20 | 0.10 | - | - | 0.05 | 0.05 | 0.20 |
| Lipase | 0.40 | 0.20 | 0.30 | 0.10 | 0.20 | - | - |
| Polishing enzyme | - | 0.04 | - | - | - | 0.004 | - |
| Extracellular-polymer-degrading enzyme | 0.05 | 0.03 | 0.01 | 0.03 | 0.03 | 0.003 | 0.003 |
| Dispersin B | - | - | - | 0.05 | 0.03 | 0.001 | 0.001 |
| Acid Violet 50 | 0.05 | - | - | - | - | - | 0.005 |
| Direct Violet 9 | - | - | - | - | - | 0.05 | - |
| Violet DD | - | 0.035 | 0.02 | 0.037 | 0.04 | - | - |
| Water insoluble plant fiber | 0.2 | - | - | - | 1.2 | - | - |
| Dye control agent | - | 0.3 | - | 0.5 | - | 0.3 | - |
| Alkoxylated polyaryl/ polyalkyl phenol | - | - | 1.2 | - | - | - | 3.1 |
| Water, dyes & minors | Balance | | | | | | |
| pH | 8.2 | | | | | | |

Based on total cleaning and/or treatment composition weight. Enzyme levels are reported as raw material.

### Examples 8 to 18: Unit Dose Compositions.

These examples provide various formulations for unit dose laundry detergents. Compositions 8 to 12 comprise a single unit dose compartment. The film used to encapsulate the compositions is polyvinyl alcohol-based film.

| **Ingredients** | **8** | **9** | **10** | **11** | **12** |
|---|---|---|---|---|---|
| | **% weight** | | | | |
| LAS | 19.09 | 16.76 | 8.59 | 6.56 | 3.44 |
| AE3S | 1.91 | 0.74 | 0.18 | 0.46 | 0.07 |
| AE7 | 14.00 | 17.50 | 26.33 | 28.08 | 31.59 |
| Citric Acid | 0.6 | 0.6 | 0.6 | 0.6 | 0.6 |
| C12-15 Fatty Acid | 14.8 | 14.8 | 14.8 | 14.8 | 14.8 |
| Polymer 3 | 4.0 | 4.0 | 4.0 | 4.0 | 4.0 |
| Chelant 2 | 1.2 | 1.2 | 1.2 | 1.2 | 1.2 |
| Optical Brightener 1 | 0.20 | 0.25 | 0.01 | 0.01 | 0.50 |
| Optical Brightener 2 | 0.20 | - | 0.25 | 0.03 | 0.01 |
| Optical Brightener 3 | 0.18 | 0.09 | 0.30 | 0.01 | - |
| DTI 1 | 0.10 | - | 0.20 | 0.01 | 0.05 |
| DTI 2 | - | 0.10 | 0.20 | 0.25 | 0.05 |
| Glycerol | 6.1 | 6.1 | 6.1 | 6.1 | 6.1 |
| Monoethanol amine | 8.0 | 8.0 | 8.0 | 8.0 | 8.0 |
| Tri-isopropanol amine | - | - | 2.0 | - | - |
| Tri-ethanol amine | - | 2.0 | - | - | - |
| Cumene sulfonate | - | - | - | - | 2.0 |
| Protease | 0.80 | 0.60 | 0.07 | 1.00 | 1.50 |
| Mannanase | 0.07 | 0.05 | 0.05 | 0.10 | 0.01 |
| Amylase 1 | 0.20 | 0.11 | 0.30 | 0.50 | 0.05 |
| Amylase 4, preferably from c) and/or h) | 0.11 | 0.20 | 0.10 | - | 0.50 |
| Isoamylase | 0.10 | 0.10 | 0.20 | 0.20 | 0.10 |
| Polishing enzyme | 0.005 | 0.05 | - | - | - |
| Extracellular-polymer-degrading enzyme | 0.005 | 0.05 | 0.005 | 0.010 | 0.005 |
| Dispersin B | 0.010 | 0.05 | 0.005 | 0.005 | - |
| Cyclohexyl dimethanol | - | - | - | 2.0 | - |
| Acid violet 50 | 0.03 | 0.02 | | | |
| Violet DD | | | 0.01 | 0.05 | 0.02 |
| Structurant | 0.14 | 0.14 | 0.14 | 0.14 | 0.14 |
| Perfume | 1.9 | 1.9 | 1.9 | 1.9 | 1.9 |
| Water insoluble plant fiber | - | 0.3 | | - | 0.1 |
| Dye control agent | - | - | 0.2 | - | - |
| Alkoxylated polyaryl/ polyalkyl phenol | 0.3 | - | - | 0.9 | - |
| Water and miscellaneous | To 100% | | | | |
| pH | 7.5-8.2 | | | | |

Based on total cleaning and/or treatment composition weight. Enzyme levels are reported as raw material.

In the following examples the unit dose has three compartments, but similar compositions can be made with two, four or five compartments. The film used to encapsulate the compartments is polyvinyl alcohol.

| **Base compositions Ingredients** | **13** | | **14** | | **15** | **16** |
|---|---|---|---|---|---|---|
| | **% weight** | | | | | |
| HLAS | 26.82 | | 16.35 | | 7.50 | 3.34 |
| AE7 | 17.88 | | 16.35 | | 22.50 | 30.06 |
| Citric Acid | 0.5 | | 0.7 | | 0.6 | 0.5 |
| C12-15 Fatty acid | 16.4 | | 6.0 | | 11.0 | 13.0 |
| Polymer 1 | 2.9 | | 0.1 | | - | - |
| Polymer 3 | 1.1 | | 5.1 | | 2.5 | 4.2 |
| Cationic cellulose polymer | - | | - | | 0.3 | 0.5 |
| Polymer 6 | - | | 1.5 | | 0.3 | 0.2 |
| Chelant 2 | 1.1 | | 2.0 | | 0.6 | 1.5 |
| Optical Brightener 1 | 0.20 | | 0.25 | | 0.01 | 0.005 |
| Optical Brightener 3 | 0.18 | | 0.09 | | 0.30 | 0.005 |
| DTI 1 | 0.1 | | - | | 0.2 | - |
| DTI 2 | - | | 0.1 | | 0.2 | - |
| Glycerol | 5.3 | | 5.0 | | 5.0 | 4.2 |
| Monoethano lamine | 10.0 | | 8.1 | | 8.4 | 7.6 |
| Polyethylene glycol | - | | - | | 2.5 | 3.0 |
| Potassium sulfite | 0.2 | | 0.3 | | 0.5 | 0.7 |
| Protease | 0.80 | | 0.60 | | 0.40 | 0.80 |
| Amylase 4, preferably from c) and/or h) | 0.20 | | 0.20 | | 0.20 | 0.30 |
| Isoamylase | 0.20 | | 0.05 | | 0.05 | 0.20 |
| Polishing enzyme | - | | - | | 0.005 | 0.005 |
| Extracellular-polymer-degrading enzyme | 0.05 | | 0.010 | | 0.005 | 0.005 |
| Dispersin B | - | | 0.010 | | 0.010 | 0.010 |
| MgCl₂ | 0.2 | | 0.2 | | 0.1 | 0.3 |
| Structurant | 0.2 | | 0.1 | | 0.2 | 0.2 |
| Acid Violet 50 | 0.04 | | 0.03 | | 0.05 | 0.03 |
| Perfume / encapsulates | 0.10 | | 0.30 | | 0.01 | 0.05 |
| Water-insoluble plant fiber | - | | - | | 0.4 | - |
| Dye control agent | - | | 0.6 | | - | 1.2 |
| Alkoxylated polyaryl/ polyalkyl phenol | 1.1 | | - | | - | - |
| Solvents and misc. | To 100% | | | | | |
| pH | 7.0-8.2 | | | | | |

| **Finishing compositions** | | **17** | | | **18** | |
|---|---|---|---|---|---|---|
| Compartment | A | B | C | A | B | C |
| Volume of each compartment | 40 ml | 5 ml | 5 ml | 40 ml | 5 ml | 5 ml |

| **Ingredients** | Active material in Wt.% | | | | | |
|---|---|---|---|---|---|---|
| Perfume | 1.6 | 1.6 | 1.6 | 1.6 | 1.6 | 1.6 |
| Violet DD | 0 | 0.006 | 0 | 0 | 0.004 | - |
| TiO2 | - | - | 0.1 | - | | 0.1 |
| Sodium Sulfite | 0.4 | 0.4 | 0.4 | 0.3 | 0.3 | 0.3 |
| Polymer 5 | - | | | 2 | - | - |
| Hydrogenated castor oil | 0.14 | 0.14 | 0.14 | 0.14 | 0.14 | 0.14 |
| Base Composition 13, 14, 15 or 16 | Add to 100% | | | | | |

Based on total cleaning and/or treatment composition weight, enzyme levels are reported as raw material.

### Examples 19 to 24: Granular laundry detergent compositions for hand washing or washing machines, typically top-loading washing machines.

| **Ingredient** | **19** | **20** | **21** | **22** | **23** | **24** |
|---|---|---|---|---|---|---|
| | **% weight** | | | | | |
| LAS | 11.33 | 10.81 | 7.04 | 4.20 | 3.92 | 2.29 |
| Quaternary ammonium | 0.70 | 0.20 | 1.00 | 0.60 | - | - |
| AE3S | 0.51 | 0.49 | 0.32 | - | 0.08 | 0.10 |
| AE7 | 8.36 | 11.50 | 12.54 | 11.20 | 16.00 | 21.51 |
| Sodium Tripolyphosphate | 5.0 | - | 4.0 | 9.0 | 2.0 | - |
| Zeolite A | - | 1.0 | - | 1.0 | 4.0 | 1.0 |
| Sodium silicate 1.6R | 7.0 | 5.0 | 2.0 | 3.0 | 3.0 | 5.0 |
| Sodium carbonate | 20.0 | 17.0 | 23.0 | 14.0 | 14.0 | 16.0 |
| Polyacrylate MW 4500 | 1.0 | 0.6 | 1.0 | 1.0 | 1.5 | 1.0 |
| Polymer 6 | 0.1 | 0.2 | - | - | 0.1 | - |
| Carboxymethyl cellulose | 1.0 | 0.3 | 1.0 | 1.0 | 1.0 | 1.0 |
| Acid Violet 50 | 0.05 | - | 0.02 | - | 0.04 | - |
| Violet DD | - | 0.03 | - | 0.03 | - | 0.03 |
| Protease 2 | 0.10 | 0.10 | 0.10 | 0.10 | - | 0.10 |
| Amylase 2, 3 or 4 | 0.03 | 0.01 | 0.03 | 0.03 | 0.03 | 0.30 |
| Isoamylase | 0.03 | 0.07 | 0.10 | 0.03 | 0.10 | 0.03 |
| Lipase | 0.03 | 0.07 | 0.30 | 0.10 | 0.07 | 0.40 |
| Polishing enzyme | 0.002 | - | 0.05 | - | 0.02 | - |
| Extracellular-polymer -degrading enzyme | 0.001 | 0.001 | 0.01 | 0.05 | 0.002 | 0.02 |
| Dispersin B | 0.001 | 0.001 | 0.05 | - | 0.001 | - |
| Optical Brightener 1 | 0.200 | 0.001 | 0.300 | 0.650 | 0.050 | 0.001 |
| Optical Brightener 2 | 0.060 | - | 0.650 | 0.180 | 0.200 | 0.060 |
| Optical Brightener 3 | 0.100 | 0.060 | 0.050 | - | 0.030 | 0.300 |
| Chelant 1 | 0.60 | 0.80 | 0.60 | 0.25 | 0.60 | 0.60 |
| DTI 1 | 0.32 | 0.15 | 0.15 | - | 0.10 | 0.10 |
| DTI 2 | 0.32 | 0.15 | 0.30 | 0.30 | 0.10 | 0.20 |
| Sodium Percarbonate | - | 5.2 | 0.1 | - | - | - |
| Sodium Perborate | 4.4 | - | 3.85 | 2.09 | 0.78 | 3.63 |
| Nonanoyloxybenzensulfonate | 1.9 | 0.0 | 1.66 | 0.0 | 0.33 | 0.75 |
| Tetraacetylehtylenediamine | 0.58 | 1.2 | 0.51 | 0.0 | 0.015 | 0.28 |
| Photobleach | 0.0030 | 0.0 | 0.0012 | 0.0030 | 0.0021 | - |
| S-ACMC | 0.1 | 0.0 | 0.0 | 0.0 | 0.06 | 0.0 |
| Water-insoluble plant fiber | - | - | 2.4 | - | - | - |
| Dye control agent | - | - | - | 2.2 | - | - |
| Alkoxylated polyaryl/ polyalkyl phenol | 1.9 | - | - | - | 2.2 | - |
| Acyl hydrazone | - | 0.7 | - | - | - | 0.6 |
| Sulfate/Moisture | Balance | | | | | |

### Examples 25-30: Granular laundry detergent compositions typically for front-loading automatic washing machines.

| **Ingredient** | **25** | **26** | **27** | **28** | **29** | **30** |
|---|---|---|---|---|---|---|
| | **% weight** | | | | | |
| LAS | 6.08 | 5.05 | 4.27 | 3.24 | 2.30 | 1.09 |
| AE3S | - | 0.90 | 0.21 | 0.18 | - | 0.06 |
| AS | 0.34 | - | - | - | - | - |
| AE7 | 4.28 | 5.95 | 6.72 | 7.98 | 9.20 | 10.35 |
| Quaternary ammonium | 0.5 | - | - | 0.3 | - | - |
| Crystalline layered silicate | 4.1 | - | 4.8 | - | - | - |
| Zeolite A | 5.0 | - | 2.0 | - | 2.0 | 2.0 |
| Citric acid | 3.0 | 4.0 | 3.0 | 4.0 | 2.5 | 3.0 |
| Sodium carbonate | 11.0 | 17.0 | 12.0 | 15.0 | 18.0 | 18.0 |
| Sodium silicate 2R | 0.08 | - | 0.11 | - | - | - |
| Optical Brightener 1 | - | 0.25 | 0.05 | 0.01 | 0.10 | 0.02 |
| Optical Brightener 2 | - | - | 0.25 | 0.20 | 0.01 | 0.08 |
| Optical Brightener 3 | - | 0.06 | 0.04 | 0.15 | - | 0.05 |
| DTI 1 | 0.08 | - | 0.04 | - | 0.10 | 0.01 |
| DTI 2 | 0.08 | - | 0.04 | 0.10 | 0.10 | 0.02 |
| Soil release agent | 0.75 | 0.72 | 0.71 | 0.72 | - | - |
| Acrylic /maleic acid copolymer | 1.1 | 3.7 | 1.0 | 3.7 | 2.6 | 3.8 |
| Carboxymethyl cellulose | 0.2 | 1.4 | 0.2 | 1.4 | 1.0 | 0.5 |
| Protease 3 | 0.20 | 0.20 | 0.30 | 0.15 | 0.12 | 0.13 |
| Amylase 3 or 4 | 0.20 | 0.15 | 0.20 | 0.30 | 0.15 | 0.15 |
| Lipase | 0.05 | 0.15 | 0.10 | - | - | - |
| Amylase 2 | 0.03 | 0.07 | - | - | 0.05 | 0.05 |
| Cellulase 2 | - | - | - | - | 0.10 | 0.10 |
| Isoamylase | 0.20 | 0.07 | 0.10 | 0.20 | 0.07 | 0.20 |
| Polishing enzyme | 0.003 | 0.005 | 0.020 | - | - | - |
| Extracellular-polymer-degrading enzyme | 0.002 | 0.010 | 0.020 | 0.020 | 0.010 | 0.003 |
| Dispersin B | 0.002 | 0.010 | 0.020 | 0.020 | 0.010 | 0.002 |
| Tetraacetylehtylenediamine | 3.6 | 4.0 | 3.6 | 4.0 | 2.2 | 1.4 |
| Sodium percabonate | 13.0 | 13.2 | 13.0 | 13.2 | 16.0 | 14.0 |
| Chelant 3 | - | 0.2 | - | 0.2 | - | 0.2 |
| Chelant 2 | 0.2 | - | 0.2 | - | 0.2 | 0.2 |
| MgSO₄ | - | 0.42 | - | 0.42 | - | 0.4 |
| Perfume | 0.5 | 0.6 | 0.5 | 0.6 | 0.6 | 0.6 |
| Suds suppressor agglomerate | 0.05 | 0.10 | 0.05 | 0.10 | 0.06 | 0.05 |
| Soap | 0.45 | 0.45 | 0.45 | 0.45 | - | - |
| Acid Violet 50 | 0.04 | - | 0.05 | - | 0.04 | - |
| Violet DD | - | 0.04 | - | 0.05 | - | 0.04 |
| S-ACMC | 0.01 | 0.01 | - | 0.01 | - | - |
| Direct Violet 9 (active) | - | - | 0.0001 | 0.0001 | - | - |
| Water-insoluble plant fiber | 1.23 | - | - | - | - | - |
| Dye control agent | - | 0.1 | - | - | - | - |
| Alkoxylated polyaryl/polyalkyl phenol | - | - | 0.81 | - | - | - |
| Acyl hydrazone | - | - | - | 0.3 | 0.06 | 0.3 |
| Sulfate/ Water & Miscellaneous | Balance | | | | | |

- Acyl hydrazone: Acyl hydrazone in accordance with the present disclosure, for example 4-(2-(2-((2-hydroxyphenylmethyl)methylene)-hydrazinyl)-2-oxoethyl)-4-methylchloride suppled as Tinocat® LT (BASF)
- AE1.8S: is C₁₂₋₁₅ alkyl ethoxy (1.8) sulfate
- AE3S: is C₁₂₋₁₅ alkyl ethoxy (3) sulfate
- AE7: is C₁₂₋₁₃ alcohol ethoxylate, with an average degree of ethoxylation of 7
- AE8: is C₁₂₋₁₃ alcohol ethoxylate, with an average degree of ethoxylation of 8
- AE9: is C₁₂₋₁₃ alcohol ethoxylate, with an average degree of ethoxylation of 9
- Alkoxylated polyaryl/: is alkoxylated polyaryl/polyalkyl phenol in accordance with the
- polyalkyl phenol: present disclosure, for example Emulsogen® TS160, Hostapal® BV conc., Sapogenat® T110 or Sapogenat® T139, all from Clariant
- Amylase 1: is Stainzyme®, 15 mg active/g
- Amylase 2: is Natalase®, 29 mg active/g
- Amylase 3: is Stainzyme Plus®, 20 mg active/g,
- Amylase 4: Amylase as described in any of a) to 1) herein.
- Isoamylase: is SEQ ID NO. 1 according to the invention or a variant thereof having glycogen-debranching activity and at least 60% or at least 70% or at least 80% or at least 90% identity with SEQ ID NO:1.
- AS: is C₁₂₋₁₄ alkylsulfate
- Cellulase 2: is Celluclean™, 15.6 mg active/g
- Xyloglucanase: is Whitezyme®, 20mg active/g
- Chelant 1: is diethylene triamine pentaacetic acid
- Chelant 2: is 1-hydroxyethane 1,1-diphosphonic acid
- Chelant 3: is sodium salt of ethylenediamine-N,N'-disuccinic acid, (S,S) isomer (EDDS)
- Dispersin B: is a glycoside hydrolase, reported as 1000mg active/g
- DTI 1: is poly(4-vinylpyridine-1-oxide) (such as Chromabond S-403E®),
- DTI 2: is poly(1-vinylpyrrolidone-co-1-vinylimidazole) (such as Sokalan HP56®).
- Dye control agent: Dye control agent in accordance with the present disclosure, for example Suparex® O.IN (M1), Nylofixan® P (M2), Nylofixan® PM (M3), or Nylofixan® HF (M4)
- HSAS: is mid-branched alkyl sulfate as disclosed in US 6,020,303 and US6,060,443
- LAS: is linear alkylbenzenesulfonate having an average aliphatic carbon chain length C₉-C₁₅ (HLAS is acid form).
- Lipase: is Lipex®, 18 mg active/g
- Mannanase: is Mannaway®, 25 mg active/g
- Optical Brightener 1: is disodium 4,4'-bis{[4-anilino-6-morpholino-s-triazin-2-yl]-amino}-2,2'-stilbenedisulfonate
- Optical Brightener 2: is disodium 4,4'-bis-(2-sulfostyryl)biphenyl (sodium salt)
- Optical Brightener 3: is Optiblanc SPL10® from 3V Sigma
- Perfume encapsulate: is a core-shell melamine formaldehyde perfume microcapsules.
- Photobleach: is a sulfonated zinc phthalocyanine
- Polishing enzyme: is Para-nitrobenzyl esterase, reported as 1000mg active/g
- Polymer 1: is bis((C₂H₅O)(C₂H₄O)n)(CH₃)-N⁺-CₓH₂ₓ-N⁺-(CH₃)-bis((C₂H₅O)(C₂H₄O)n), wherein n = 20-30,x = 3 to 8 or sulphated or sulfonated variants thereof
- Polymer 2: is ethoxylated (EO₁₅) tetraethylene pentamine
- Polymer 3: is ethoxylated polyethylenimine
- Polymer 4: is ethoxylated hexamethylene diamine, Baxxodur® ECX 210 from BASF SE, Baxxodur® EC 301 from BASF SE, or a polyetheramine comprising 1 mol 2-butyl-2-ethyl-1,3-propanediol + 5.0 mole propylene oxide, aminated.
- Polymer 5: is Acusol 305, provided by Rohm&Haas
- Polymer 6: is a polyethylene glycol polymer grafted with vinyl acetate side chains, provided by BASF.
- Protease: is Purafect Prime®, 40.6 mg active/g
- Protease 2: is Savinase®, 32.89 mg active/g
- Protease 3: is Purafect®, 84 mg active/g
- Quaternary ammonium: is C₁₂₋₁₄ Dimethylhydroxyethyl ammonium chloride
- S-ACMC: is Reactive Blue 19 Azo-CM-Cellulose provided by Megazyme
- Soil release agent: is Repel-o-tex® SF2
- Structurant: is Hydrogenated Castor Oil
- Violet DD: is a thiophene azo dye provided by Milliken
- Water insoluble plant fiber: is water insoluble plant fiber in accordance with the present disclosure, for example Herbacel AQ+ Type N, supplied by Herbafood Ingredients GmbH, Werder, Germany.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cleaning composition comprising a glycogen-debranching enzyme having at least 60%, preferably at least 70% identity to SEQ ID NO:1; b) a second amylase, said second amylase having activity in breaking alpha-1,4- glycosidic bonds; and c) a cleaning adjunct.

2. A cleaning composition according to claim 1, wherein the glycogen-debranching enzyme has at least 70%, or at least 80%, or at least 90% or at least 95% identity with the amino acid sequence shown in SEQ ID NO:1.

3. A cleaning composition according to claim 1 or claim 2 wherein the glycogen-debranching enzyme is selected Glycoside Hydrolase Family 13.

4. A cleaning composition according to any preceding claim wherein the glycogen-debranching enzyme is obtainable from *Pseudomonas, Corynebacterium glutamicum* or *E. coli,* preferably *E. coli.*

5. A cleaning composition according to any preceding claim wherein the second amylase has at least 80% identity with the wild type amylase from Bacillus SP722.

6. A cleaning composition according to any preceding claim, wherein the cleaning adjunct comprises a bleach system.

7. A cleaning composition according to any preceding claim wherein the cleaning adjunct comprises a non-soap anionic surfactant or mixture of non-soap anionic surfactants.

8. A cleaning composition according to claim 7 wherein the non-soap anionic surfactant is part of a surfactant system comprising non-soap anionic surfactant and nonionic surfactant, preferably the weight ratio of non-soap anionic surfactant to nonionic surfactant being from 100:1 to 1:1.

9. A cleaning composition according to any preceding claim wherein the non-soap anionic surfactant comprises linear alkyl benzene sulphonate, optionally in combination with optionally alkoxylated alkyl sulfate surfactant and mixtures thereof.

10. A cleaning composition according to any preceding claim which provides a pH less than 9, preferably from 7 to 8.9 for a 1 wt% solution in deionised water.

11. A cleaning composition according to any preceding claim wherein the composition comprises a bleach agent, preferably comprising a peroxygen source and bleach catalyst and/or bleach activator.

12. A method of making a cleaning composition according to any preceding claim comprising mixing the glycogen-debranching enzyme, second amylase and cleaning adjunct.

13. A method of cleaning a surface, preferably a textile, comprising mixing the cleaning composition according to any of claims 1 to 11 with water to form an aqueous liquor and contacting a surface, preferably a textile, with the aqueous liquor in a washing step.

14. A method according to claim 13 wherein the surface is contacted with the aqueous liquor for from 1 to 50 minutes, preferably from 5 to 40 minutes, most preferably from 5 to 30 minutes.

15. A method according to claim 13 or claim 14 wherein the temperature of the aqueous liquor is from 5 to 40°C, preferably from 5 to 30°C, preferably from 5 to 20°C.


SEQUENCE LISTING

<110>  The Procter & Gamble Company

<120>  Cleaning compositions

<130>  CM04954FM

<160>  15

<170>  PatentIn version 3.5

<210>  1
<211>  657
<212>  PRT
<213>  E. coli

<400>  1

Met Thr Gln Leu Ala Ile Gly Lys Pro Ala Pro Leu Gly Ala His Tyr
1               5                   10                  15

Asp Gly Gln Gly Val Asn Phe Thr Leu Phe Ser Ala His Ala Glu Arg
            20                  25                  30

Val Glu Leu Cys Val Phe Asp Ala Asn Gly Gln Glu His Arg Tyr Asp
        35                  40                  45

Leu Pro Gly His Ser Gly Asp Ile Trp His Gly Tyr Leu Pro Asp Ala
    50                  55                  60

Arg Pro Gly Leu Arg Tyr Gly Tyr Arg Val His Gly Pro Trp Gln Pro
65                  70                  75                  80

Ala Glu Gly His Arg Phe Asn Pro Ala Lys Leu Leu Ile Asp Pro Cys
                85                  90                  95

Ala Arg Gln Ile Asp Gly Glu Phe Lys Asp Asn Pro Leu Leu His Ala
            100                 105                 110

Gly His Asn Glu Pro Asp Tyr Arg Asp Asn Ala Ala Ile Ala Pro Lys
        115                 120                 125

Cys Val Val Val Val Asp His Tyr Asp Trp Glu Asp Asp Ala Pro Pro
    130                 135                 140

Arg Thr Pro Trp Gly Ser Thr Ile Ile Tyr Glu Ala His Val Lys Gly
145                 150                 155                 160

Leu Thr Tyr Leu His Pro Glu Ile Pro Val Glu Ile Arg Gly Thr Tyr
                165                 170                 175

Lys Ala Leu Gly His Pro Val Met Ile Asn Tyr Leu Lys Gln Leu Gly
        180                 185                 190

Ile Thr Ala Leu Glu Leu Leu Pro Val Ala Gln Phe Ala Ser Glu Pro
    195                 200                 205

Arg Leu Gln Arg Met Gly Leu Ser Asn Tyr Trp Gly Tyr Asn Pro Val
    210                 215                 220

Ala Met Phe Ala Leu His Pro Ala Tyr Ala Cys Ser Pro Glu Thr Ala
225                 230                 235                 240

Leu Asp Glu Phe Arg Asp Ala Ile Lys Ala Leu His Lys Ala Gly Ile
                245                 250                 255

Glu Val Ile Leu Asp Ile Val Leu Asn His Ser Ala Glu Leu Asp Leu
            260                 265                 270

Asp Gly Pro Leu Phe Ser Leu Arg Gly Ile Asp Asn Arg Ser Tyr Tyr
        275                 280                 285

Trp Ile Arg Glu Asp Gly Asp Tyr His Asn Trp Thr Gly Cys Gly Asn
    290                 295                 300

Thr Leu Asn Leu Ser His Pro Ala Val Val Asp Tyr Ala Ser Ala Cys
305                 310                 315                 320

Leu Arg Tyr Trp Val Glu Thr Cys His Val Asp Gly Phe Arg Phe Asp
                325                 330                 335

Leu Ala Ala Val Met Gly Arg Thr Pro Glu Phe Arg Gln Asp Ala Pro
            340                 345                 350

Leu Phe Thr Ala Ile Gln Asn Cys Pro Val Leu Ser Gln Val Lys Leu
        355                 360                 365

Ile Ala Glu Pro Trp Asp Ile Ala Pro Gly Gly Tyr Gln Val Gly Asn
    370                 375                 380

Phe Pro Pro Leu Phe Ala Glu Trp Asn Asp His Phe Arg Asp Ala Ala
385                 390                 395                 400

Arg Arg Phe Trp Leu His Tyr Asp Leu Pro Leu Gly Ala Phe Ala Gly
                405                 410                 415

Arg Phe Ala Ala Ser Ser Asp Val Phe Lys Arg Asn Gly Arg Leu Pro
            420                 425                 430

Ser Ala Ala Ile Asn Leu Val Thr Ala His Asp Gly Phe Thr Leu Arg
        435                 440                 445

Asp Cys Val Cys Phe Asn His Lys His Asn Glu Ala Asn Gly Glu Glu
    450                 455                 460

Asn Arg Asp Gly Thr Asn Asn Asn Tyr Ser Asn Asn His Gly Lys Glu
465                 470                 475                 480

Gly Leu Gly Gly Ser Leu Asp Leu Val Glu Arg Arg Arg Asp Ser Ile
                485                 490                 495

His Ala Leu Leu Thr Thr Leu Leu Leu Ser Gln Gly Thr Pro Met Leu
            500                 505                 510

Leu Ala Gly Asp Glu His Gly His Ser Gln His Gly Asn Asn Asn Ala
        515                 520                 525

Tyr Cys Gln Asp Asn Gln Leu Thr Trp Leu Asp Trp Ser Gln Ala Ser
    530                 535                 540

Ser Gly Leu Thr Ala Phe Thr Ala Ala Leu Ile His Leu Arg Lys Arg
545                 550                 555                 560

Ile Pro Ala Leu Val Glu Asn Arg Trp Trp Glu Glu Gly Asp Gly Asn
                565                 570                 575

Val Arg Trp Leu Asn Arg Tyr Ala Gln Pro Leu Ser Thr Asp Glu Trp
            580                 585                 590

Gln Asn Gly Pro Lys Gln Leu Gln Ile Leu Leu Ser Asp Arg Phe Leu
        595                 600                 605

Ile Ala Ile Asn Ala Thr Leu Glu Val Thr Glu Ile Val Leu Pro Ala
    610                 615                 620

Gly Glu Trp His Ala Ile Pro Pro Phe Ala Gly Glu Asp Asn Pro Val
625                 630                 635                 640

Ile Thr Ala Val Trp Gln Gly Pro Ala His Gly Leu Cys Val Phe Gln
                645                 650                 655

Arg

<210>  2
<211>  463

<212>  PRT
<213>  Streptomyces davawensis

<400>  2

Asp Ala Thr Ile Val Ile Asn Pro Gly Thr Arg Tyr Gly Thr Trp Glu
1               5                   10                  15

Gly Trp Gly Thr Ser Leu Ala Trp Trp Gly Asn Val Phe Gly Thr Arg
            20                  25                  30

Asp Asp Phe Ala Asp Leu Phe Phe Thr Thr Lys Ser Val Thr Tyr Asn
        35                  40                  45

Gly Thr Ser Leu Pro Gly Leu Gly Leu Asn Ile Ala Arg Tyr Asn Leu
    50                  55                  60

Gly Ala Cys Ser Trp Asn Ala Val Asn Gly Glu Thr Met Val Lys Ser
65                  70                  75                  80

Pro Asn Ile Pro Ala Phe Lys Gln Ile Glu Gly Phe Trp Gln Asp Trp
                85                  90                  95

Asn Asn Glu Asp Pro Thr Ser Ser Ala Trp Asp Trp Thr Ala Asp Ala
            100                 105                 110

Thr Gln Arg Ala Met Leu Val Lys Ala Thr Gln Arg Gly Ala Val Thr
        115                 120                 125

Glu Leu Phe Ala Asn Ser Pro Met Trp Trp Met Cys Tyr Asn His Asn
    130                 135                 140

Pro Ser Gly Ala Ala Asp Gly Gly Asn Asn Leu Gln Thr Trp Asn Tyr
145                 150                 155                 160

Arg Gln His Ala Ser His Leu Ala Ala Val Ala Leu Tyr Ala Arg Thr
                165                 170                 175

Asn Trp Gly Val Asn Phe Ala Thr Val Asp Pro Phe Asn Glu Pro Ala
            180                 185                 190

Ser Ser Trp Trp Thr Ala Ser Gly Thr Gln Glu Gly Cys His Leu Asp
        195                 200                 205

Pro Ala Val Gln Ala Ala Val Leu Pro Tyr Met Arg Ser Glu Leu Asp
    210                 215                 220

Lys Arg Gly Leu Thr Gly Val Arg Ile Ser Ala Ser Asp Glu Thr Asn
225                 230                 235                 240

Tyr Asp Thr Ala Arg Ser Thr Trp Ser Ser Phe Gly Ser Ala Thr Lys
                245                 250                 255

Ala Leu Val Ser Gln Val Asn Val His Gly Tyr Gln Gly Thr Gly Gly
            260                 265                 270

Arg Arg Asp Leu Leu Tyr Thr Asp Val Val Thr Thr Ser Gly Lys Lys
        275                 280                 285

Leu Trp Asn Ser Glu Thr Gly Asp Ser Asp Gly Thr Gly Leu Ser Met
    290                 295                 300

Ala Arg Asn Leu Cys Tyr Asp Phe Arg Trp Leu His Pro Thr Ala Trp
305                 310                 315                 320

Cys Tyr Trp Gln Val Met Asp Pro Ser Thr Gly Trp Ala Met Ile Ala
                325                 330                 335

Tyr Asp Ala Asn Thr Leu Gln Pro Thr Thr Val Gln Pro Lys Tyr Tyr
            340                 345                 350

Val Met Ala Gln Phe Ser Arg His Ile Arg Pro Gly Met Thr Ile Leu
        355                 360                 365

Asp Thr Gly Val Ser Phe Ala Ala Ala Ala Tyr Asp Ala Ser Ala Arg
    370                 375                 380

Arg Leu Val Leu Val Ala Val Asn Thr Ser Thr Ser Pro Gln Thr Phe
385                 390                 395                 400

Thr Phe Asp Leu Ser Arg Phe Thr Thr Val Thr Gly Gly Ser Gly Gly
                405                 410                 415

Leu Val Pro Arg Trp Asn Thr Val Thr Gly Gly Gly Asp Met Tyr Arg
            420                 425                 430

Ala Tyr Thr Asn Thr Tyr Val Thr Gly Lys Ser Val Ser Ala Thr Phe
        435                 440                 445

Ala Ala Gly Ser Val Gln Thr Leu Gln Val Asp Gly Val Thr Thr
    450                 455                 460

<210>  3
<211>  464
<212>  PRT
<213>  Streptomyces avermitilis

<400> 3

Asp Ala Thr Ile Ala Val Asn Pro Ser Thr Thr Tyr Gly Lys Trp Glu
1               5                   10                  15

Gly Trp Gly Thr Ser Leu Ala Trp Trp Ala Asn Val Phe Gly Ala Arg
            20                  25                  30

Asp Asp Phe Ala Asp Leu Phe Phe Thr Thr Lys Ser Val Thr Tyr Asn
        35                  40                  45

Gly Arg Thr Leu Pro Gly Leu Gly Leu Asn Ile Ala Arg Tyr Asn Leu
    50                  55                  60

Gly Ala Cys Ser Trp Asn Ser Val Ser Gly Glu Ser Met Val Ala Ser
65                  70                  75                  80

Ala Asn Ile Pro Ala Phe Lys Gln Ile Glu Gly Tyr Trp Gln Asp Trp
                85                  90                  95

Asn Asn Glu Asp Pro Thr Ser Ser Ala Trp Lys Trp Thr Ala Asp Ala
            100                 105                 110

Ala Gln Arg Thr Met Leu Val Lys Ala Thr Ala Arg Gly Ala Thr Thr
        115                 120                 125

Glu Leu Phe Ala Asn Ser Pro Met Trp Trp Met Cys Leu Asn His Asn
    130                 135                 140

Pro Ser Gly Ala Ser Gly Gly Gly Asn Asn Leu Gln Ser Trp Asn Tyr
145                 150                 155                 160

Arg Gln His Ala Ser His Leu Ala Ala Val Ala Leu Tyr Ala Lys Ser
                165                 170                 175

Asn Trp Gly Val Asn Phe Ala Thr Val Asp Pro Phe Asn Glu Pro Ser
            180                 185                 190

Ser Ser Trp Trp Thr Ala Thr Gly Thr Gln Glu Gly Cys His Met Asp
        195                 200                 205

Ala Ser Val Gln Ala Ala Val Leu Pro Tyr Leu Arg Ser Glu Leu Asp
    210                 215                 220

Arg Arg Gly Leu Thr Gly Thr Lys Ile Ser Ala Ser Asp Glu Thr Ser
225                 230                 235                 240

Tyr Asp Leu Ala Arg Thr Thr Trp Gly Ser Phe Gly Ser Ser Thr Lys

245                 250                 255

Ala Leu Val Asn Arg Val Asn Val His Gly Tyr Gln Gly Ser Gly Gly
            260                 265                 270

Arg Arg Asp Leu Leu Tyr Thr Asp Val Val Thr Thr Ala Gly Lys Ala
        275                 280                 285

Leu Trp Asn Ser Glu Thr Gly Asp Ser Asp Gly Thr Gly Leu Thr Leu
    290                 295                 300

Ala Ser Asn Leu Cys Leu Asp Phe Arg Trp Leu His Pro Thr Ala Trp
305                 310                 315                 320

Val Tyr Trp Gln Val Met Asp Pro Ser Ser Gly Trp Ala Met Ile Ala
                325                 330                 335

Tyr Asp Ala Ser Thr Leu Gln Pro Gly Ala Val Gln Thr Lys Tyr Tyr
            340                 345                 350

Val Met Ala Gln Phe Ser Arg His Ile Arg Ala Gly Met Thr Ile Val
        355                 360                 365

Asp Thr Gly Val Gly Tyr Ala Ala Ala Ala Tyr Asp Ala Thr Ala Arg
    370                 375                 380

Arg Leu Val Ile Val Ala Val Asn Thr Ser Thr Ser Ala Gln Thr Leu
385                 390                 395                 400

Thr Phe Asp Leu Ser Arg Phe Ser Thr Val Thr Gly Gly Thr Gly Gly
                405                 410                 415

Leu Val Arg Arg Trp Asn Thr Val Thr Gly Gly Gly Gly Asp Leu Tyr
            420                 425                 430

Ala Ala His Ser Asp Thr Tyr Leu Ser Gly Lys Ser Leu Ser Val Pro
        435                 440                 445

Phe Ala Ala Gly Ala Val Gln Thr Leu Glu Val Asp Gly Val Thr Val
    450                 455                 460

<210>  4
<211>  458
<212>  PRT
<213>  Trichoderma harzianum

<400>  4

Asp Thr Thr Leu Ser Ile Asp Pro Thr Ser Asn Trp Gly Thr Trp Glu

1               5                   10                  15

Gly Trp Gly Val Ser Leu Ala Trp Trp Ala Lys Ala Phe Gly Asn Arg
            20                  25                  30

Asp Asp Leu Ala Asn Val Phe Phe Thr Arg Asn Asn Gln Val Ile Asn
        35                  40                  45

Gly Gln Asn Leu Pro Gly Leu Gly Phe Asn Ile Ala Arg Tyr Asn Ala
    50                  55                  60

Gly Ala Cys Ser Thr Asn Thr Tyr Asn Gly Ser Ser Met Val Val Ser
65                  70                  75                  80

Ser Ser Ile Lys Pro Ser Arg Gln Val Asp Gly Tyr Trp Leu Asp Trp
                85                  90                  95

Ala Ser Thr Asp Pro Ala Ser Ser Ser Trp Asn Trp Asn Val Asp Ala
            100                 105                 110

Asn Gln Arg Ala Met Leu Gln Lys Ala Lys Ala Asn Gly Ala Asn Ile
        115                 120                 125

Phe Glu Leu Phe Ser Asn Ser Pro Met Trp Trp Met Cys Leu Asn His
    130                 135                 140

Asn Pro Ser Gly Ser Gly Ser Ser Asp Asn Leu Gln Ser Trp Asn Tyr
145                 150                 155                 160

Gln Asn His Ala Val Tyr Leu Ala Asn Ile Ala Gln His Ala Gln Gln
                165                 170                 175

Asn Trp Gly Ile Gln Phe Gln Ser Val Glu Ala Phe Asn Glu Pro Ser
            180                 185                 190

Ser Gly Trp Gly Pro Thr Gly Thr Gln Glu Gly Cys His Phe Ala Val
        195                 200                 205

Ser Thr Met Ala Thr Val Ile Gly Tyr Leu Asn Thr Glu Leu Ala Gln
    210                 215                 220

Arg Gly Leu Ser Ser Phe Ile Ser Ala Ser Asp Glu Thr Ser Tyr Asp
225                 230                 235                 240

Leu Ala Ile Ser Thr Trp Gln Gly Leu Gly Ser Ser Ala Gln Asn Ala
                245                 250                 255

Val Lys Arg Val Asn Val His Gly Tyr Gln Gly Gly Gly Gly Arg Arg
            260                 265                 270

Asp Thr Leu Tyr Ser Leu Val Ser Gln Ala Gly Lys Arg Leu Trp Asn
        275                 280                 285

Ser Glu Tyr Gly Asp Ala Asp Ala Ser Gly Lys Ser Met Tyr Thr Asn
    290                 295                 300

Leu Leu Leu Asp Phe Thr Trp Leu His Pro Thr Ala Trp Val Tyr Trp
305                 310                 315                 320

Gln Ala Ile Asp Gly Ser Gly Trp Gly Leu Ile Val Gly Asp Asn Asp
                325                 330                 335

Gln Leu Thr Leu Ser Ser Ala Ser Thr Lys Tyr Phe Val Leu Ala Gln
            340                 345                 350

Leu Thr Arg His Ile Arg Pro Gly Met Gln Ile Leu Thr Thr Pro Asp
        355                 360                 365

Gly Asn Thr Val Ala Ala Tyr Asp Ser Gly Ser Gln Lys Leu Val Ile
    370                 375                 380

Val Ala Ala Asn Trp Gly Ser Ala Gln Thr Ile Thr Phe Asp Leu Thr
385                 390                 395                 400

Arg Ala Lys Thr Ala Gly Ser Asn Gly Ala Thr Val Pro Arg Trp Ser
                405                 410                 415

Thr Gln Thr Ser Gly Gly Asp Gln Tyr Lys Ser Tyr Ser Asp Thr Lys
            420                 425                 430

Ile Asn Asn Gly Lys Phe Ser Val Ser Phe Ser Thr Gly Gln Val Gln
        435                 440                 445

Thr Phe Glu Ile Ser Gly Val Val Leu Lys
    450                 455

<210>  5
<211>  109
<212>  PRT
<213>  Bacillus licheniformis

<400>  5

Ala Arg Tyr Asp Asp Val Leu Tyr Phe Pro Ala Ser Arg Tyr Pro Glu
1               5                   10                  15

Thr Gly Ala His Ile Ser Asp Ala Ile Lys Ala Gly His Ala Asp Val
            20                  25                  30

Cys Thr Ile Glu Arg Ser Gly Ala Asp Lys Arg Arg Gln Glu Ser Leu
        35                  40                  45

Lys Gly Ile Pro Thr Lys Pro Gly Phe Asp Arg Asp Glu Trp Pro Met
    50                  55                  60

Ala Met Cys Glu Glu Gly Gly Lys Gly Ala Ser Val Arg Tyr Val Ser
65                  70                  75                  80

Ser Ser Asp Asn Arg Gly Ala Gly Ser Trp Val Gly Asn Arg Leu Asn
                85                  90                  95

Gly Tyr Ala Asp Gly Thr Arg Ile Leu Phe Ile Val Gln
            100                 105

<210>  6
<211>  109
<212>  PRT
<213>  Bacillus subtilis

<400>  6

Ala Ser Ser Tyr Asp Lys Val Leu Tyr Phe Pro Leu Ser Arg Tyr Pro
1               5                   10                  15

Glu Thr Gly Ser His Ile Arg Asp Ala Ile Ala Glu Gly His Pro Asp
            20                  25                  30

Ile Cys Thr Ile Asp Asp Gly Ala Asp Lys Arg Arg Glu Glu Ser Leu
        35                  40                  45

Lys Gly Ile Pro Thr Lys Pro Gly Tyr Asp Arg Asp Glu Trp Pro Met
    50                  55                  60

Ala Val Cys Glu Glu Gly Gly Ala Gly Ala Asp Val Arg Tyr Val Thr
65                  70                  75                  80

Pro Ser Asp Asn Arg Gly Ala Gly Ser Trp Val Gly Asn Gln Met Ser
                85                  90                  95

Ser Tyr Pro Asp Gly Thr Arg Val Leu Phe Ile Val Gln
            100                 105

<210>  7
<211>  109
<212>  PRT
<213>  Bacillus licheniformis

<400>  7

Ala Arg Tyr Asp Asp Ile Leu Tyr Phe Pro Ala Ser Arg Tyr Pro Glu
1               5                   10                  15

Thr Gly Ala His Ile Ser Asp Ala Ile Lys Ala Gly His Ser Asp Val
            20                  25                  30

Cys Thr Ile Glu Arg Ser Gly Ala Asp Lys Arg Arg Gln Glu Ser Leu
        35                  40                  45

Lys Gly Ile Pro Thr Lys Pro Gly Phe Asp Arg Asp Glu Trp Pro Met
    50                  55                  60

Ala Met Cys Glu Glu Gly Gly Lys Gly Ala Ser Val Arg Tyr Val Ser
65                  70                  75                  80

Ser Ser Asp Asn Arg Gly Ala Gly Ser Trp Val Gly Asn Arg Leu Ser
                85                  90                  95

Gly Phe Ala Asp Gly Thr Arg Ile Leu Phe Ile Val Gln
            100                 105

<210>  8
<211>  204
<212>  PRT
<213>  Aspergillus oryzae

<400>  8

Lys Thr Gly Ser Gly Asp Ser Gln Ser Asp Pro Ile Lys Ala Asp Leu
1               5                   10                  15

Glu Val Lys Gly Gln Ser Ala Leu Pro Phe Asp Val Asp Cys Trp Ala
            20                  25                  30

Ile Leu Cys Lys Gly Ala Pro Asn Val Leu Gln Arg Val Asn Glu Lys
        35                  40                  45

Thr Lys Asn Ser Asn Arg Asp Arg Ser Gly Ala Asn Lys Gly Pro Phe
    50                  55                  60

Lys Asp Pro Gln Lys Trp Gly Ile Lys Ala Leu Pro Pro Lys Asn Pro
65                  70                  75                  80

Ser Trp Ser Ala Gln Asp Phe Lys Ser Pro Glu Glu Tyr Ala Phe Ala
                85                  90                  95

Ser Ser Leu Gln Gly Gly Thr Asn Ala Ile Leu Ala Pro Val Asn Leu

100                 105                 110

Ala Ser Gln Asn Ser Gln Gly Gly Val Leu Asn Gly Phe Tyr Ser Ala
        115                 120                 125

Asn Lys Val Ala Gln Phe Asp Pro Ser Lys Pro Gln Gln Thr Lys Gly
    130                 135                 140

Thr Trp Phe Gln Ile Thr Lys Phe Thr Gly Ala Ala Gly Pro Tyr Cys
145                 150                 155                 160

Lys Ala Leu Gly Ser Asn Asp Lys Ser Val Cys Asp Lys Asn Lys Asn
                165                 170                 175

Ile Ala Gly Asp Trp Gly Phe Asp Pro Ala Lys Trp Ala Tyr Gln Tyr
            180                 185                 190

Asp Glu Lys Asn Asn Lys Phe Asn Tyr Val Gly Lys
        195                 200

<210>  9
<211>  188
<212>  PRT
<213>  Trichoderma harzianum

<400>  9

Ala Pro Ala Pro Met Pro Thr Pro Pro Gly Ile Pro Thr Glu Ser Ser
1               5                   10                  15

Ala Arg Thr Gln Leu Ala Gly Leu Thr Val Ala Val Ala Gly Ser Gly
            20                  25                  30

Thr Gly Tyr Ser Arg Asp Leu Phe Pro Thr Trp Asp Ala Ile Ser Gly
        35                  40                  45

Asn Cys Asn Ala Arg Glu Tyr Val Leu Lys Arg Asp Gly Glu Gly Val
    50                  55                  60

Gln Val Asn Asn Ala Cys Glu Ser Gln Ser Gly Thr Trp Ile Ser Pro
65                  70                  75                  80

Tyr Asp Asn Ala Ser Phe Thr Asn Ala Ser Ser Leu Asp Ile Asp His
                85                  90                  95

Met Val Pro Leu Lys Asn Ala Trp Ile Ser Gly Ala Ser Ser Trp Thr
            100                 105                 110

Thr Ala Gln Arg Glu Ala Leu Ala Asn Asp Val Ser Arg Pro Gln Leu

115                 120                 125

Trp Ala Val Ser Ala Ser Ala Asn Arg Ser Lys Gly Asp Arg Ser Pro
    130                 135                 140

Asp Gln Trp Lys Pro Pro Leu Thr Ser Phe Tyr Cys Thr Tyr Ala Lys
145                 150                 155                 160

Ser Trp Ile Asp Val Lys Ser Phe Tyr Lys Leu Thr Ile Thr Ser Ala
                165                 170                 175

Glu Lys Thr Ala Leu Ser Ser Met Leu Asp Thr Cys
            180                 185

<210>  10
<211>  361
<212>  PRT
<213>  Aggregatibacter actinomycetemcomitans

<400>  10

Asn Cys Cys Val Lys Gly Asn Ser Ile Tyr Pro Gln Lys Thr Ser Thr
1               5                   10                  15

Lys Gln Thr Gly Leu Met Leu Asp Ile Ala Arg His Phe Tyr Ser Pro
            20                  25                  30

Glu Val Ile Lys Ser Phe Ile Asp Thr Ile Ser Leu Ser Gly Gly Asn
        35                  40                  45

Phe Leu His Leu His Phe Ser Asp His Glu Asn Tyr Ala Ile Glu Ser
    50                  55                  60

His Leu Leu Asn Gln Arg Ala Glu Asn Ala Val Gln Gly Lys Asp Gly
65                  70                  75                  80

Ile Tyr Ile Asn Pro Tyr Thr Gly Lys Pro Phe Leu Ser Tyr Arg Gln
                85                  90                  95

Leu Asp Asp Ile Lys Ala Tyr Ala Lys Ala Lys Gly Ile Glu Leu Ile
            100                 105                 110

Pro Glu Leu Asp Ser Pro Asn His Met Thr Ala Ile Phe Lys Leu Val
        115                 120                 125

Gln Lys Asp Arg Gly Val Lys Tyr Leu Gln Gly Leu Lys Ser Arg Gln
    130                 135                 140

Val Asp Asp Glu Ile Asp Ile Thr Asn Ala Asp Ser Ile Thr Phe Met

145                 150                 155                 160

Gln Ser Leu Met Ser Glu Val Ile Asp Ile Phe Gly Asp Thr Ser Gln
                165                 170                 175

His Phe His Ile Gly Gly Asp Glu Phe Gly Tyr Ser Val Glu Ser Asn
            180                 185                 190

His Glu Phe Ile Thr Tyr Ala Asn Lys Leu Ser Tyr Phe Leu Glu Lys
        195                 200                 205

Lys Gly Leu Lys Thr Arg Met Trp Asn Asp Gly Leu Ile Lys Asn Thr
    210                 215                 220

Phe Glu Gln Ile Asn Pro Asn Ile Glu Ile Thr Tyr Trp Ser Tyr Asp
225                 230                 235                 240

Gly Asp Thr Gln Asp Lys Asn Glu Ala Ala Glu Arg Arg Asp Met Arg
                245                 250                 255

Val Ser Leu Pro Glu Leu Leu Ala Lys Gly Phe Thr Val Leu Asn Tyr
            260                 265                 270

Asn Ser Tyr Tyr Leu Tyr Ile Val Pro Lys Ala Ser Pro Thr Phe Ser
        275                 280                 285

Gln Asp Ala Ala Phe Ala Ala Lys Asp Val Ile Lys Asn Trp Asp Leu
    290                 295                 300

Gly Val Trp Asp Gly Arg Asn Thr Lys Asn Arg Val Gln Asn Thr His
305                 310                 315                 320

Glu Ile Ala Gly Ala Ala Leu Ser Ile Trp Gly Glu Asp Ala Lys Ala
                325                 330                 335

Leu Lys Asp Glu Thr Ile Gln Lys Asn Thr Lys Ser Leu Leu Glu Ala
            340                 345                 350

Val Ile His Lys Thr Asn Gly Asp Glu
        355                 360

<210>  11
<211>  541
<212>  PRT
<213>  Ascobolus stictoideus

<400>  11

Gln Thr Tyr Thr Leu Glu Ala Glu Ala Gly Thr Leu Thr Gly Val Thr

1               5                   10                  15

Val Met Asn Glu Ile Ala Gly Phe Ser Gly Thr Gly Tyr Val Gly Gly
            20                  25                  30

Trp Asp Glu Asp Ala Asp Thr Val Ser Leu Thr Phe Thr Ser Asp Ala
        35                  40                  45

Thr Lys Leu Tyr Asp Val Lys Ile Arg Tyr Ser Gly Pro Tyr Gly Ser
    50                  55                  60

Lys Tyr Thr Arg Ile Ser Tyr Asn Gly Ala Thr Gly Gly Asp Ile Ser
65                  70                  75                  80

Leu Pro Glu Thr Thr Glu Trp Ala Thr Val Asn Ala Gly Gln Ala Leu
                85                  90                  95

Leu Asn Ala Gly Ser Asn Thr Ile Lys Leu His Asn Asn Trp Gly Trp
            100                 105                 110

Tyr Leu Ile Asp Ala Val Ile Leu Thr Pro Ser Val Pro Arg Pro Pro
        115                 120                 125

His Gln Val Thr Asp Ala Leu Val Asn Thr Asn Ser Asn Ala Val Thr
    130                 135                 140

Lys Gln Leu Met Lys Phe Leu Val Ser Lys Tyr His Lys Ala Tyr Ile
145                 150                 155                 160

Thr Gly Gln Gln Glu Leu His Ala His Gln Trp Val Glu Lys Asn Val
                165                 170                 175

Gly Lys Ser Pro Ala Ile Leu Gly Leu Asp Phe Met Asp Tyr Ser Pro
            180                 185                 190

Ser Arg Val Glu Phe Gly Thr Thr Ser Gln Ala Val Glu Gln Ala Ile
        195                 200                 205

Asp Phe Asp Lys Arg Gly Gly Ile Val Thr Phe Ala Trp His Trp Asn
    210                 215                 220

Ala Pro Ser Gly Leu Ile Asn Thr Pro Gly Ser Glu Trp Trp Arg Gly
225                 230                 235                 240

Phe Tyr Thr Glu His Thr Thr Phe Asp Val Ala Ala Ala Leu Gln Asn
                245                 250                 255

Thr Thr Asn Ala Asn Tyr Asn Leu Leu Ile Arg Asp Ile Asp Ala Ile
            260                 265                 270

Ala Val Gln Leu Lys Arg Leu Gln Thr Ala Gly Val Pro Val Leu Trp
        275                 280                 285

Arg Pro Leu His Glu Ala Glu Gly Gly Trp Phe Trp Trp Gly Ala Lys
    290                 295                 300

Gly Pro Glu Pro Ala Lys Lys Leu Tyr Lys Ile Leu Tyr Asp Arg Leu
305                 310                 315                 320

Thr Asn Tyr His Lys Leu Asn Asn Leu Ile Trp Val Trp Asn Ser Val
                325                 330                 335

Ala Lys Asp Trp Tyr Pro Gly Asp Glu Ile Val Asp Val Leu Ser Phe
            340                 345                 350

Asp Ser Tyr Pro Ala Gln Pro Gly Asp His Gly Pro Val Ser Ala Gln
        355                 360                 365

Tyr Asn Ala Leu Val Glu Leu Gly Lys Asp Lys Lys Leu Ile Ala Ala
    370                 375                 380

Thr Glu Val Gly Thr Ile Pro Asp Pro Asp Leu Met Gln Leu Tyr Glu
385                 390                 395                 400

Ser Tyr Trp Ser Phe Phe Val Thr Trp Glu Gly Glu Phe Ile Glu Asn
                405                 410                 415

Gly Val His Asn Ser Leu Glu Phe Leu Lys Lys Leu Tyr Asn Asn Ser
            420                 425                 430

Phe Val Leu Asn Leu Asp Thr Ile Gln Gly Trp Lys Asn Gly Ala Gly
        435                 440                 445

Ser Ser Thr Thr Thr Val Lys Ser Thr Thr Thr Thr Pro Thr Thr Thr
    450                 455                 460

Ile Lys Ser Thr Thr Thr Thr Pro Val Thr Thr Pro Thr Thr Val Lys
465                 470                 475                 480

Thr Thr Thr Thr Pro Thr Thr Thr Ala Thr Thr Val Lys Ser Thr Thr
                485                 490                 495

Thr Thr Ala Gly Pro Thr Pro Thr Ala Val Ala Gly Arg Trp Gln Gln
            500                 505                 510

Cys Gly Gly Ile Gly Phe Thr Gly Pro Thr Thr Cys Glu Ala Gly Thr
        515                 520                 525

Thr Cys Asn Val Leu Asn Pro Tyr Tyr Ser Gln Cys Leu
    530                 535                 540

<210>  12
<211>  526
<212>  PRT
<213>  Chaetomium virescens

<400>  12

Pro Arg Asp Pro Gly Ala Thr Ala Arg Thr Phe Glu Ala Glu Asp Ala
1               5                   10                  15

Thr Leu Ala Gly Thr Asn Val Asp Thr Ala Leu Ser Gly Phe Thr Gly
            20                  25                  30

Thr Gly Tyr Val Thr Gly Phe Asp Gln Ala Ala Asp Lys Val Thr Phe
        35                  40                  45

Thr Val Asp Ser Ala Ser Thr Glu Leu Tyr Asp Leu Ser Ile Arg Val
    50                  55                  60

Ala Ala Ile Tyr Gly Asp Lys Arg Thr Ser Val Val Leu Asn Gly Gly
65                  70                  75                  80

Ala Ser Ser Glu Val Tyr Phe Pro Ala Gly Glu Thr Trp Thr Asn Val
                85                  90                  95

Ala Ala Gly Gln Leu Leu Leu Asn Gln Gly Ser Asn Thr Ile Asp Ile
            100                 105                 110

Val Ser Asn Trp Gly Trp Tyr Leu Ile Asp Ser Ile Thr Leu Thr Pro
        115                 120                 125

Ser Thr Pro Arg Pro Ala His Gln Ile Asn Glu Ala Pro Val Asn Ala
    130                 135                 140

Ala Ala Asp Lys Asn Ala Lys Ala Leu Tyr Ser Tyr Leu Arg Ser Ile
145                 150                 155                 160

Tyr Gly Lys Lys Ile Leu Ser Gly Gln Gln Glu Leu Ser Leu Ser Asn
                165                 170                 175

Trp Ile Ala Gln Gln Thr Gly Lys Thr Pro Ala Leu Val Ser Val Asp
            180                 185                 190

Leu Met Asp Tyr Ser Pro Ser Arg Val Glu Arg Gly Thr Val Gly Thr
        195                 200                 205

Ala Val Glu Glu Ala Ile Gln His His Asn Arg Gly Gly Ile Val Ser
    210                 215                 220

Val Leu Trp His Trp Asn Ala Pro Thr Gly Leu Tyr Asp Thr Glu Glu
225                 230                 235                 240

His Arg Trp Trp Ser Gly Phe Tyr Thr Ser Ala Thr Asp Phe Asp Val
                245                 250                 255

Ala Ala Ala Leu Ser Ser Thr Thr Asn Ala Asn Tyr Thr Leu Leu Ile
            260                 265                 270

Arg Asp Ile Asp Ala Ile Ala Val Gln Leu Lys Arg Leu Gln Ser Ala
        275                 280                 285

Gly Val Pro Val Leu Phe Arg Pro Leu His Glu Ala Glu Gly Gly Trp
    290                 295                 300

Phe Trp Trp Gly Ala Lys Gly Pro Glu Pro Ala Lys Lys Leu Trp Gly
305                 310                 315                 320

Ile Leu Tyr Asp Arg Val Thr Asn His His Gln Ile Asn Asn Leu Leu
                325                 330                 335

Trp Val Trp Asn Ser Ile Leu Pro Glu Trp Tyr Pro Gly Asp Ala Thr
            340                 345                 350

Val Asp Ile Leu Ser Ala Asp Val Tyr Ala Gln Gly Asn Gly Pro Met
        355                 360                 365

Ser Thr Gln Tyr Asn Gln Leu Ile Glu Leu Gly Lys Asp Lys Lys Met
    370                 375                 380

Ile Ala Ala Ala Glu Val Gly Ala Ala Pro Leu Pro Asp Leu Leu Gln
385                 390                 395                 400

Ala Tyr Glu Ala His Trp Leu Trp Phe Thr Val Trp Gly Asp Ser Phe
                405                 410                 415

Ile Asn Asn Ala Asp Trp Asn Ser Leu Asp Thr Leu Lys Lys Val Tyr
            420                 425                 430

Thr Ser Asp Tyr Val Leu Thr Leu Asp Glu Ile Gln Gly Trp Gln Gly
        435                 440                 445

Ser Thr Pro Ser Ala Thr Thr Thr Ser Ser Thr Thr Thr Pro Ser Ala
    450                 455                 460

Thr Thr Thr Thr Thr Thr Pro Ser Thr Thr Ala Thr Thr Ala Thr Pro
465                 470                 475                 480

Ser Ala Thr Thr Thr Ala Ser Pro Val Thr Tyr Ala Glu His Trp Gly
                485                 490                 495

Gln Cys Ala Gly Lys Gly Trp Thr Gly Pro Thr Thr Cys Arg Pro Pro
            500                 505                 510

Tyr Thr Cys Lys Tyr Gln Asn Asp Trp Tyr Ser Gln Cys Leu
        515                 520                 525

<210>  13
<211>  452
<212>  PRT
<213>  Preussia aemulans

<400>  13

Gln Thr Val Ile Tyr Gln Ala Glu Gln Ala Lys Leu Ser Gly Val Thr
1               5                   10                  15

Val Glu Phe Ser Ile Ile Lys Gln Val Val Gly Thr Gly Tyr Val Glu
            20                  25                  30

Gly Phe Asp Glu Ser Thr Asp Ser Ile Thr Phe Thr Val Glu Ser Thr
        35                  40                  45

Thr Ala Ala Leu Tyr Asp Leu Ala Leu Thr Tyr Asn Gly Pro Tyr Gly
    50                  55                  60

Asp Lys Tyr Thr Asn Val Val Leu Asn Asn Ala Ala Gly Ser Gln Val
65                  70                  75                  80

Ser Leu Pro Ala Thr Thr Ala Trp Thr Thr Val Pro Ala Gly Gln Val
                85                  90                  95

Leu Leu Asn Ala Gly Ala Asn Thr Ile Gln Ile Gln Asn Asn Trp Gly
            100                 105                 110

Trp Tyr Leu Val Asp Ser Ile Ser Leu Lys Pro Ala Ala Thr Arg Gly
        115                 120                 125

Ala His Gln Ile Thr Thr Lys Pro Val Asn Lys Asn Ala Asn Ser Asp
    130                 135                 140

Ala Lys Ala Leu Leu Lys Tyr Leu Gly Ser Ile Tyr Gly Lys Lys Ile
145                 150                 155                 160

Leu Ser Gly Gln Gln Asp Leu Ser Ser Leu Asp Trp Val Thr Lys Asn
                165                 170                 175

Val Gly Lys Thr Pro Ala Val Leu Gly Leu Asp Thr Met Asp Tyr Ser
            180                 185                 190

Glu Ser Arg Lys Ser Arg Gly Ala Val Ser Thr Asp Val Asp Lys Ala
        195                 200                 205

Ile Ala Phe Ala Lys Lys Gly Gly Ile Val Thr Phe Cys Trp His Trp
    210                 215                 220

Gly Ala Pro Thr Gly Leu Phe Asp Ser Ala Ala Gln Pro Trp Tyr Arg
225                 230                 235                 240

Gly Phe Tyr Thr Asp Ala Thr Asp Phe Asn Ile Glu Thr Ala Leu Lys
                245                 250                 255

Asp Thr Thr Asn Ala Asn Tyr Thr Leu Leu Met Lys Asp Ile Asp Thr
            260                 265                 270

Ile Ala Val Gln Leu Lys Lys Leu Gln Asp Ala Gly Val Pro Val Ile
        275                 280                 285

Trp Arg Pro Leu His Glu Ala Glu Gly Gly Trp Phe Trp Trp Gly Ala
    290                 295                 300

Lys Gly Pro Glu Pro Ala Lys Lys Leu Trp Lys Ile Met Tyr Asp Arg
305                 310                 315                 320

Leu Thr Asn Gln His Gly Leu Asn Asn Leu Val Trp Thr Trp Asn Ser
                325                 330                 335

Val Ala Pro Asn Trp Tyr Pro Gly Asp Asp Thr Val Asp Ile Val Ser
            340                 345                 350

Ala Asp Thr Tyr Ser Gln Gly Asp His Gly Pro Ile Ser Ala Thr Tyr
        355                 360                 365

Asn Asn Leu Leu Ala Leu Thr Asn Asp Thr Lys Ile Ile Ala Ala Ala
    370                 375                 380

Glu Ile Gly Ser Val Met Glu Pro Ala Gln Leu Gln Ala Tyr Gln Ala

385                 390                 395                 400

Asp Trp Val Tyr Phe Cys Val Trp Ser Gly Glu Phe Ile Asp Gly Gly
                405                 410                 415

Val Trp Asn Ser Leu Asp Phe Leu Lys Lys Val Tyr Asn Asp Pro Tyr
            420                 425                 430

Val Leu Thr Leu Asp Glu Ile Gln Gly Trp Lys Thr Ala Arg Gly Lys
        435                 440                 445

Pro Arg Val Ser
    450

<210>  14
<211>  312
<212>  PRT
<213>  Yunnania penicillata

<400>  14

Ala Pro Ser Thr Thr Pro Val Asn Glu Lys Ala Thr Asp Ala Ala Lys
1               5                   10                  15

Asn Leu Leu Ser Tyr Leu Val Glu Gln Ala Ala Asn Gly Val Thr Leu
            20                  25                  30

Ser Gly Gln Gln Asp Leu Glu Ser Ala Gln Trp Val Ser Asp Asn Val
        35                  40                  45

Gly Lys Trp Pro Ala Ile Leu Gly Ile Asp Phe Met Asp Tyr Ser Pro
    50                  55                  60

Ser Arg Val Glu Tyr Gly Ala Val Gly Ser Thr Val Pro Asp Ala Ile
65                  70                  75                  80

Ser Tyr Asp Ser Asp Gly Gly Ile Val Thr Phe Cys Trp His Trp Gly
                85                  90                  95

Ser Pro Ser Gly Thr Tyr Asn Thr Thr Asp Gln Pro Trp Trp Ser Asn
            100                 105                 110

Phe Tyr Thr Glu Ala Thr Ala Phe Asp Ile Ala Ala Ala Met Asp Asp
        115                 120                 125

Pro Asp Ser Ala Asp Tyr Asn Leu Leu Val Arg Asp Ile Asp Ala Ile
    130                 135                 140

Ser Glu Leu Leu Leu Gln Leu Gln Asp Leu Asp Ile Pro Ile Leu Trp

145                 150                 155                 160

Arg Pro Leu His Glu Ala Glu Gly Gly Trp Phe Trp Trp Gly Ala Lys
                165                 170                 175

Gly Pro Glu Ala Cys Ile Ala Leu Tyr Arg Leu Met Phe Asp Arg Met
            180                 185                 190

Thr Asn His His Gly Leu Asn Asn Leu Leu Trp Val Trp Asn Ser Val
        195                 200                 205

Asp Pro Ser Trp Tyr Pro Gly Asn Asp Val Val Asp Ile Val Ser Ala
    210                 215                 220

Asp Ile Tyr Ala Asp Ala Gly Asp His Ser Pro Gln Glu Glu Thr Phe
225                 230                 235                 240

Ala Ser Leu Gln Ser Leu Thr Gly Asp Thr Lys Leu Val Ala Leu Gly
                245                 250                 255

Glu Val Gly Asn Ile Pro Asp Pro Ala Ser Thr Gly Gly Val Ala Asp
            260                 265                 270

Trp Ala Tyr Trp Val Thr Trp Asn Gly Asp Phe Ile Lys Gly Glu Asp
        275                 280                 285

Tyr Asn Pro Leu Glu Tyr Lys Lys Glu Val Phe Ser Ala Glu Asn Ile
    290                 295                 300

Ile Thr Arg Asp Glu Val Asp Val
305                 310

<210>  15
<211>  327
<212>  PRT
<213>  Myrothecium roridum

<400>  15

Gly Thr Ile Glu Asn Arg Gln Trp Leu Thr Tyr Asn Pro Val Asp Ser
1               5                   10                  15

Ala Ala Thr Thr Glu Ala Arg Ala Leu Leu Arg Tyr Ile Gln Ser Gln
            20                  25                  30

Tyr Gly Trp Arg Tyr Leu Ser Gly Gln Gln Glu Arg Ala Glu Val Gln
        35                  40                  45

Trp Leu Lys Ser Asn Ile Gly Lys Thr Pro Ala Ile Gln Gly Ser Asp

50                  55                  60

Leu Ile Asp Tyr Ser Pro Ser Arg Val Ser Tyr Gly Ala Thr Ser Thr
65                  70                  75                  80

Ala Val Glu Asp Ala Ile Ala Phe Asp Arg Gln Gly Gly Ile Val Thr
                85                  90                  95

Phe Thr Trp His Trp Asn Ala Pro Asn Cys Leu Tyr Asn Ser Ala Asp
            100                 105                 110

Gln Pro Trp Tyr Phe Gly Phe Tyr Thr Lys Ala Thr Cys Phe Asn Ile
        115                 120                 125

Gln Ala Ala Leu Ala Gln Gly Ser Asn Gly Ala Asp Tyr Lys Leu Leu
    130                 135                 140

Ile Arg Asp Ile Asp Ala Ile Ala Val Gln Leu Lys Arg Leu Arg Asp
145                 150                 155                 160

Ala Lys Val Pro Ile Leu Phe Arg Pro Leu His Glu Pro Asp Gly Ala
                165                 170                 175

Trp Phe Trp Trp Gly Ala Lys Gly Ser Gly Pro Phe Lys Gln Leu Trp
            180                 185                 190

Asp Ile Leu Tyr Asp Arg Leu Thr Lys Tyr His Gly Leu His Asn Met
        195                 200                 205

Leu Trp Val Cys Asn Thr Glu Lys Ser Asp Trp Tyr Pro Gly Asn Asn
    210                 215                 220

Lys Cys Asp Ile Ala Thr Thr Asp Val Tyr Val Asn Ala Gly Asp His
225                 230                 235                 240

Ser Val Gln Lys Ser His Trp Asp Ala Leu Tyr Gly Val Ser Gly Gly
                245                 250                 255

Gln Arg Ile Leu Ala Leu Gly Glu Val Gly Val Ile Pro Asp Pro Glu
            260                 265                 270

Arg Gln Ala Ser Glu Asn Val Pro Trp Ala Tyr Trp Met Thr Trp Asn
        275                 280                 285

Gly Tyr Phe Ile Arg Asp Gly Asn Tyr Asn Ser Arg Asn Phe Leu Gln
    290                 295                 300

Ser Thr Phe Ser Asn Ala Arg Val Val Thr Leu Asp Gly Thr Ser Pro
305                 310                 315                 320

Leu Gly Asn Trp Lys Ser Ser
                325